(19) Europäisches Patentamt
European Patent Office
Office européen des brevets

(11) EP 1 988 082 A1

(12) EUROPEAN PATENT APPLICATION
published in accordance with Art. 158(3) EPC

(43) Date of publication:
**05.11.2008 Bulletin 2008/45**

(21) Application number: **07714121.6**

(22) Date of filing: **07.02.2007**

(51) Int Cl.:
***C07D 235/14*** *(2006.01)* ***B01J 31/22*** *(2006.01)*
***C07D 405/06*** *(2006.01)* ***C08F 4/40*** *(2006.01)*
***C08F 12/26*** *(2006.01)* ***C08F 26/02*** *(2006.01)*
***C07F 1/08*** *(2006.01)* ***C07F 13/00*** *(2006.01)*
***C07F 15/02*** *(2006.01)* ***C07F 15/04*** *(2006.01)*
***C07F 15/06*** *(2006.01)*

(86) International application number:
**PCT/JP2007/052540**

(87) International publication number:
**WO 2007/091713 (16.08.2007 Gazette 2007/33)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **08.02.2006 JP 2006030585**

(71) Applicant: **Sumitomo Chemical Company, Limited**
**Tokyo 104-8260 (JP)**

(72) Inventors:
- **ISHIYAMA, Takeshi**
  **Tsukuba-shi, Ibaraki 305-0821 (JP)**
- **HIGASHIMURA, Hideyuki**
  **Tsukuba-shi, Ibaraki 305-0045 (JP)**

(74) Representative: **Duckworth, Timothy John et al**
**J.A. Kemp & Co.,**
**14 South Square,**
**Gray's Inn**
**London WC1R 5JJ (GB)**

# (54) POLYNUCLEAR COMPLEX AND POLYMER THEREOF

(57) To provide a multinuclear complex including at least one ligand L satisfying the following requirements (i), (ii) and (iii), and a plurality of metal atoms in a molecule:
(i) having a group having polymerizable reactive multiple bonds, and/or a ring-opening polymerizable ring,
(ii) having five or more coordination atoms bonding to the metal atoms, and
(iii) being soluble in a solvent.

EP 1 988 082 A1

## Description

### TECHNICAL FIELD

The present invention relates to a multinuclear complex containing a group having polymerizable multiple bonds and/or a ring-opening polymerizable ring, and a polymer that the multinuclear complex is polymerized. It further relates to a multinuclear complex or a polymer of the multinuclear complex suitable for a redox catalyst.

### BACKGROUND ART

As described in "Comprehensive dictionary on Chemistry" (first edition, 1994, Tokyo Kagaku Dozin Co., Ltd.), a multinuclear complex means one that contains two or more metal atoms as a central atom in one complex, and since it has an unusual, multiple reactivity based on interactions between a plurality of metal sites, it is a complex capable of being a unique catalyst for reaction, above all, it is used in an application of catalyst related to a chemical reaction involving electron transfer such as a redox catalyst (see for example, Hyomen 2003, 41 (3), 22 by Oyaizu Kenichi and Yuasa Makoto). As one example thereof, there is known an example using a binuclear manganese complex as a catalyst for decomposing hydrogen peroxide into water and oxygen (hydrogen peroxide decomposition catalyst) while suppressing the generation of free radicals (hydroxyl radical, hydroperoxy radical, etc.) in hydrogen peroxide (see A.E. Boelrijk and G.C. Dismukes, Inorg. Chem. 2000, 39, 3020). A catalyst that a metal enzyme was calcined is also known as a multinuclear complex (see for example, Japanese Unexamined Patent Publication No. 2004-217507).

### DISCLOSURE OF THE INVENTION

However, when the binuclear manganese complex disclosed so far is used as a hydrogen peroxide decomposition catalyst, stability, in particular, heat stability is not sufficient, and thus a catalyst superior in heat stability has been strongly desired.

Further, since a metal enzyme itself is not only expensive, but also is a biological material, its storage stability is poor, and a catalyst using this as a raw material was difficult to obtain production repeatability.

An object of the present invention is to provide a multinuclear complex with an excellent heat stability as well as having a unique catalyst activity, in particular, in a hydrogen peroxide decomposition catalyst, to provide a catalyst with more excellent heat stability and having a catalyst ability capable of decomposition into water and oxygen while suppressing the generation of free radicals, and further to provide a novel multinuclear complex being a precursor of the catalyst.

In order to solve the above problems, the present inventors have keenly made efforts to find a technique which can improve the distance between a plurality of metal atoms and heat stability of conformation, and as a result, have found that a polymer or copolymer obtained by polymerizing a multinuclear complex having a specific ligand has a high stability without lowering a reaction activity as a redox catalyst, and thus completed the present invention. Namely, the present invention provides multinuclear complexes described in the following [1] to [8].

[1] A multinuclear complex including at least one ligand L satisfying the following requirements (i), (ii) and (iii), and a plurality of metal atoms:

(i) having a group having polymerizable reactive multiple bonds, and/or a ring-opening polymerizable ring,
(ii) having five or more coordination atoms bonding to the metal atoms, and
(iii) being soluble in a solvent.

[2] The multinuclear complex described in [1], wherein a coordination atom of the ligand L is a nitrogen atom, an oxygen atom, a phosphorous atom or a sulfur atom.
[3] The multinuclear complex described in [1] or [2], wherein a coordination atom of the ligand L contains a nitrogen atom having a double bond with carbon.
[4] The multinuclear complex described in any one of [1] to [3], wherein the total of metal atoms contained in a molecule is 8 or less.
[5] The multinuclear complex described in any one of [1] to [4], wherein a metal atom contained in a molecule is a transition metal atom of the first transition element series.
[6] The multinuclear complex described in any one of [1] to [5], wherein two metal atoms in the multinuclear complex are coordinately bonded to the same coordination atom, or have a combination of $AM^1$ and $AM^2$ such that the minimum of a covalent bond linking $AM^1$ and $AM^2$ is 1 or more, and 4 or less, when two metal atoms selected from a plurality of metal atoms are denoted as $M^1$, $M^2$, and coordination atoms bonding to $M^1$ and $M^2$ are denoted as

$AM^1$ and $AM^2$, respectively.

[7] The multinuclear complex described in any one of [1] to [6], wherein the ligand L is one, and the metal atoms are two.

[8] The multinuclear complex described in any one of [1] to [7], wherein the molecular weight is 6000 or less.

Further, as the above ligand L, the present invention provides compounds described in the following [9] to [12] being preferable compounds, and the following [13] having the compounds as a ligand L.

[9] A compound expressed by the following formula (1):

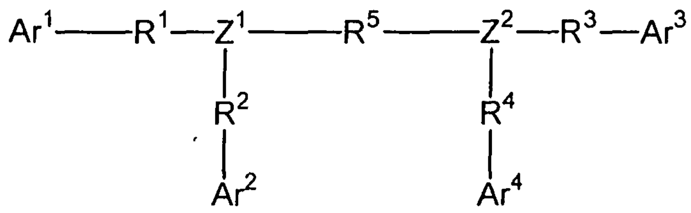

(1)

wherein $Ar^1$, $Ar^2$, $Ar^3$ and $Ar^4$ (hereinafter sometimes denoted as $Ar^1$ to $Ar^4$) each independently represents an aromatic heterocyclic group having at least one coordination atom, $R^1$, $R^2$, $R^3$, $R^4$ and $R^5$ (hereinafter sometimes denoted as $R^1$ to $R^5$) represent a divalent group, $Z^1$ and $Z^2$ each independently represents a nitrogen atom or a trivalent group; at least one of $Ar^1$ to $Ar^4$ and $R^1$ to $R^5$ has a group having polymerizable reactive multiple bonds, and/or a ring-opening polymerizable ring.

[10] The compound described in [9], expressed by the following formula (2):

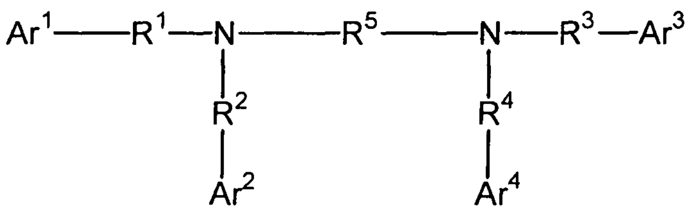

(2)

wherein $Ar^1$ to $Ar^4$, and $R^1$ to $R^5$ are the same meanings as in the formula (1), and at least one of them has a group having polymerizable reactive multiple bonds, and/or a ring-opening polymerizable ring.

[11] The compound described in [10], expressed by the following formula (3a) or (4a):

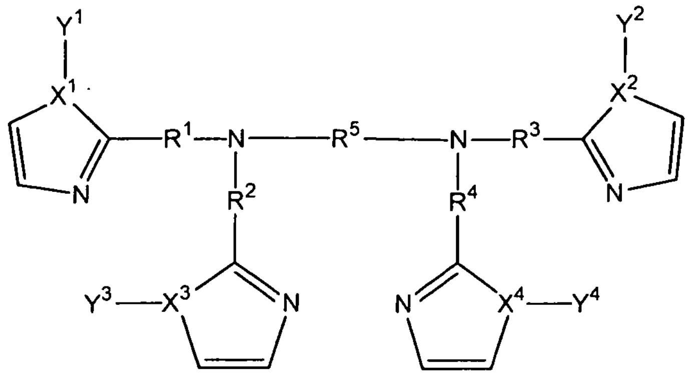

(3a)

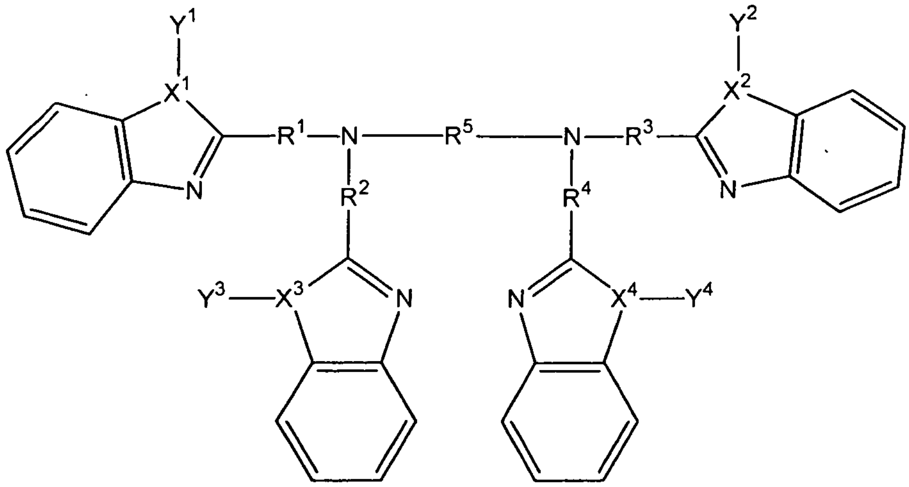

(4a)

In the formulas (3a) and (4a), $R^1$ to $R^5$ are the same definitions as in the formula (1). $X^1$, $X^2$, $X^3$ and $X^4$ (hereinafter sometimes denoted as $X^1$ to $X^4$) are selected from a nitrogen atom or CH. $Y^1$, $Y^2$, $Y^3$ and $Y^4$ (hereinafter sometimes denoted as $Y^1$ to $Y^4$) represent a hydrogen atom, an alkyl group having 1 to 50 carbon atoms, an aromatic group having 2 to 60 carbon atoms, a group having a polymerizable reactive carbon-carbon double bond or a group having a ring-opening polymerizable ring, and at least one of $Y^1$ to $Y^4$ is a group having a polymerizable reactive carbon-carbon double bond or a group having a ring-opening polymerizable ring.

[12] The compound described in [11], expressed by the following formula (3b) or (4b):

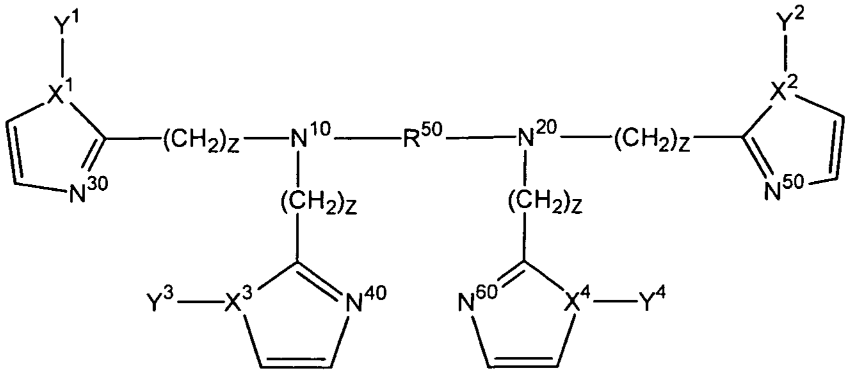

(3b)

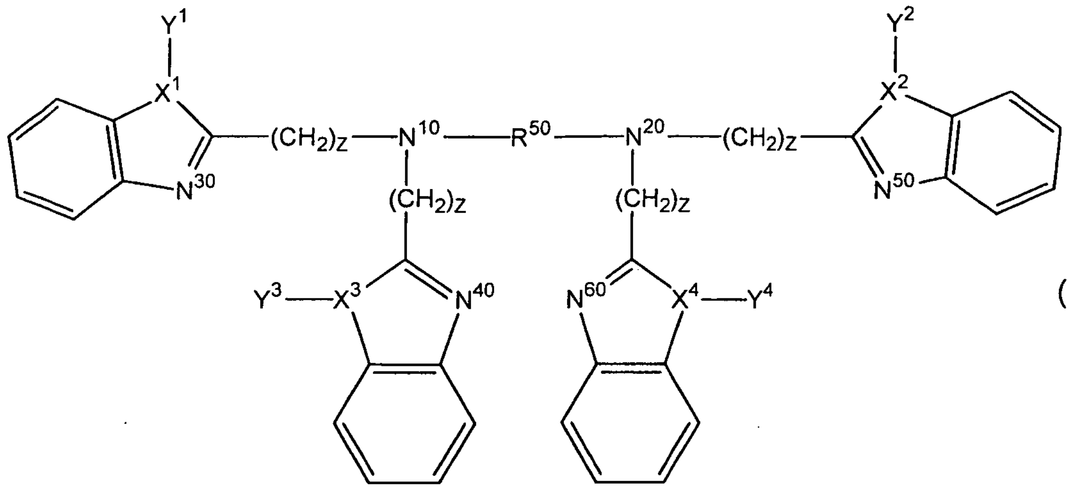

(4b)

In the formulas (3b) and (4b), $X^1$, $X^2$, $X^3$ and $X^4$ (hereinafter sometimes denoted as $X^1$ to $X^4$), and $Y^1$, $Y^2$, $Y^3$ and $Y^4$ (hereinafter sometimes denoted as $Y^1$ to $Y^4$) are the same meanings as in the formula (3a) or (4a). At least one of $Y^1$ to $Y^4$ is a group having a polymerizable reactive carbon-carbon double bond or a group having a ring-opening polymerizable ring. Z represents an integer of 1 or 2. $N^{10}$ and $N^{20}$ represent a nitrogen atom bonding with $R^{50}$, and $N^{30}$, $N^{40}$, $N^{50}$ and $N^{60}$ (hereinafter sometimes denoted as $N^{30}$ to $N^{60}$) represent a nitrogen atom in an aromatic heterocyclic group. $R^{50}$ represents a divalent group having the minimum of a covalent bond linking $N^{10}$ and $N^{20}$ of 2 or more, and 14 or less.

[13] The compound described in [12], expressed by the following formula (3c) or (4c):

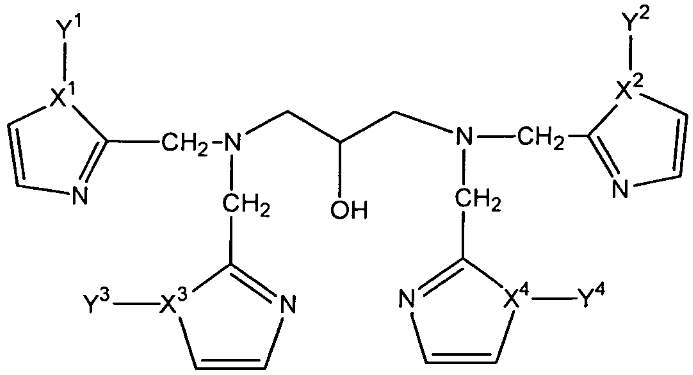

(3c)

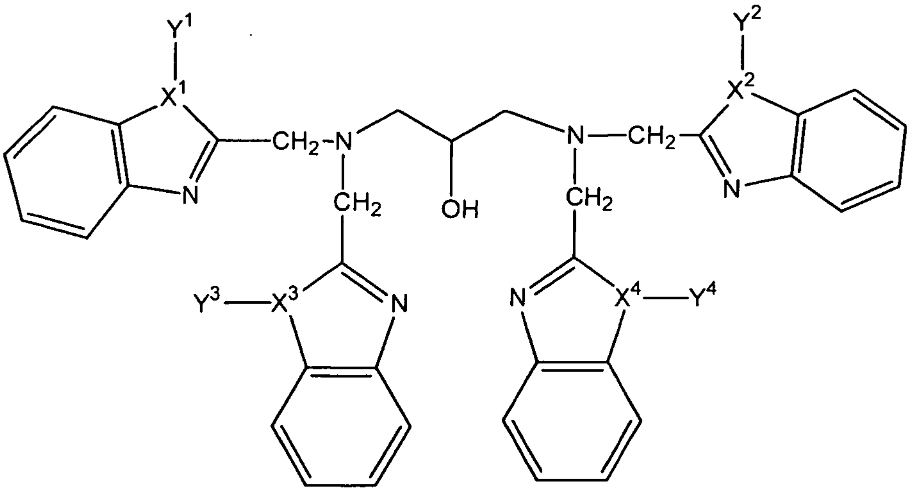

(4c)

In the formulas (3c) and (4c), $X^1$ to $X^4$, and $Y^1$to $Y^4$ are the same meanings as in the formula (3a) or (4a), and at least one of $Y^1$ to $Y^4$ is a group having a polymerizable reactive carbon-carbon double bond or a group having a ring-opening polymerizable ring.

[14] The multinuclear complex described in any one of [1] to [8], which has the compound described in any one of [9] to [13] as the ligand L.

Further, the present invention provides a (co)polymer suitable as a catalyst obtained from the multinuclear complex, and also provides an application as a catalyst. Namely, it provides:

[15] A polymer obtained by polymerizing the multinuclear complex described in any one of [1] to [8] and [14].

[16] A copolymer obtained by copolymerizing at least one kind of the multinuclear complex described in any one of [1] to [8] and [14] with a polymerizable monomer capable of copolymerizing with the multinuclear complex.

[17] A redox catalyst using the multinuclear complex described in any one of [1] to [8] and [14], the polymer described in [15], or the copolymer described in [16].

BRIEF DESCRIPTION OF THE DRAWINGS

Fig. 1 1H-NMR analysis chart of bbpr-allyl ligand in Production example 1

Fig. 2 Variation with time of the amount of oxygen generated in Examples 3 and 4

Fig. 3 1H-NMR analysis chart of bbpr-$CH_2$St ligand in Production example 3

Fig. 4 Variation with time of the amount of oxygen generated in Examples 8 and 9

BEST MODE FOR CARRYING OUT THE INVENTION

Preferable embodiments of the present invention are shown below.

The multinuclear complex of the present invention contains a plurality of metal atoms. The metal atoms may be non-charged or charged ions. The number of the metal atoms is preferably 2 or more and 8 or less, more preferably 2 or more and 4 or less, and particularly preferably 2 or 3.

The metal atoms are selected from transition metals, and they may be the same or different each other. As a specific example of the transition metals, for example, there can be exemplified transition metals of the first transition element series selected from the group consisting of scandium, titanium, vanadium, chromium, manganese, iron, cobalt, nickel, copper and zinc; yttrium, zirconium, niobium, molybdenum, ruthenium, rhodium, palladium, silver, cadmium, lanthanum, cerium, praseodymium, neodymium, promethium, samarium, europium, gadolinium, terbium, dysprosium, holmium, erbium, thulium, ytterbium, lutetium, hafnium, tantalum, tungsten, rhenium, osmium, iridium, platinum, gold, mercury, actinium, thorium, protactinium, uranium and the like.

Preferable are transition metal atoms selected from the transition metal atoms of the first transition element series; zirconium, niobium, molybdenum, ruthenium, rhodium, palladium, silver, lanthanum, cerium, praseodymium, neodymium, promethium, samarium, europium, gadolinium, terbium, dysprosium, holmium, erbium, thulium, ytterbium, lutetium, tantalum, tungsten, rhenium, osmium, iridium, platinum, and gold;

More preferable are transition metal atoms selected from the transition metal atoms of the first transition element

series; zirconium, niobium, molybdenum, ruthenium, rhodium, palladium, silver, lanthanum, cerium, samarium, europium, ytterbium, lutetium, tantalum, tungsten, rhenium, osmium, iridium, platinum and gold;

Further preferable are transition metal atoms of the first transition element series; and

Particularly preferable are transition metal atoms selected from vanadium, chromium, manganese, iron, cobalt, nickel and copper, above all, transition metal atoms selected from manganese, iron, cobalt, nickel and copper are preferable.

Next, the ligand L is explained.

The multinuclear complex of the present invention has at least one ligand L satisfying the requirements (i), (ii) and (iii). The number of the ligands L in the multinuclear complex is preferably 1 or more, and 6 or less, further preferably 1 or more, and 3 or less, especially preferably 1 or 2, and particularly preferably 1. Additionally, when there are a plurality of ligands L in the multinuclear complex, they may be the same or different.

The ligand L, as the above requirement (i), has at least one group having polymerizable reactive multiple bonds, and/or one ring-opening polymerizable ring. These groups or rings may be present in plural, and when there is a plurality, these groups or rings may be the same or different.

Herein, a group having polymerizable reactive multiple bonds is a group having a carbon-carbon double bond, a carbon-carbon triple bond and a carbon-nitrogen triple bond capable of giving addition polymerization such as radical polymerization (including thermal polymerization, photo polymerization using a photo initiator) and ion polymerization, the group having these multiple bonds may also have a divalent linking group, as the divalent linking group, there are included carboxylgroup, carbonyl group, sulfonyl group, thioxygroup, oxy group, alkylene groups having 1 to 16 carbon atoms, and divalent aromatic groups having 2 to 60 carbon atoms (including a heteroaromatic group) and the like, which may be a divalent group that these divalent groups are linked.

Specifically, examples of groups having polymerizable reactive multiple bonds include such as vinyl group, allylgroup, propenyl group, butenyl group, butadienyl group, cyclopentadienyl group, cyclohexenyl group, maleimidegroup, acroyl group, styryl group, vinylbenzyl group, ethynyl group, propynyl group, ethynylphenyl group, cyano group and isonitrile group.

Preferable are vinyl group, styryl group, vinylbenzyl group, allyl group, ethynyl group and cyano group, and more preferable are vinyl group, styryl group, vinylbenzyl group, allyl group and cyano group, further preferable are vinyl group, styryl group, vinylbenzyl group and allyl group, which are groups having a carbon-carbon double bond.

Further, an alkylene group or an aromatic group related to the divalent linking group may have a monovalent substituent, as the example, there are included hydroxyl group, mercapto group, carboxyl group, phophonic acid group, sulfonic acid group, nitro group, halogeno groups (fluoro group, chloro group, bromo group or iodo group), carbamoyl group, alkyl groups having 1 to 50 carbon atoms, aromatic groups having 2 to 60 carbon atoms (including an aromatic heterocyclic group), alkoxy groups or an alkylthio groups composed of the alkyl groups and oxy groups or thioether groups; aryloxy groups or arylthio groups composed of the aromatic groups and oxy groups or thioether groups; alkyl sulfonyl groups or aryl sulfonyl groups composed of the alkyl groups or the aromatic groups and sulfonyl groups; acyl groups or aryl carbonyl groups composed of the alkyl groups or the aromatic groups and carbonyl group; alkoxy carbonyl groups or aryloxy carbonyl groups composed of the alkyl groups or the aromatic groups and oxy carbonyl group; amino groups that may have the alkyl groups or the aromatic groups, acid amide groups that may have the alkyl groups or the aromatic groups, phosphoryl groups that may have the alkyl groups and/or the aromatic groups, thiophosphoryl groups that may have the alkyl groups and/or the aromatic groups, a silyl groups that has the alkyl groups and/or the aromatic groups and the like.

Here, as an example of the alkyl groups having 1 to 50 carbon atoms, there are included alkyl groups that can be obtained by removing one hydrogen atom from a saturate hydrocarbon compound, that is, linear alkyl groups, branched alkyl groups or cycloalkyl groups, such as methyl group, ethyl group, propyl group, isopropyl group, butyl group, isobutyl group, 2,2-dimethyl butyl group, octyl group, decyl group, dodecyl group, hexadecyl group, icosyl group, triacontyl group, pentacontyl group, cyclopentyl group, cyclohexyl group and adamantyl group.

The alkyl groups are preferably alkyl groups having 1 to 30 carbon atoms, more preferably alkyl groups having 1 to 16 carbon atoms, and particularly preferably alkyl groups having 1 to 8 carbon atoms.

Further, as an example of the aromatic groups (including an aromatic heterocyclic group), there are included aromatic groups that can be obtained by removing one hydrogen atom from an aromatic compound having carbon atoms of about 2 to 60, such as phenyl group, tolyl group, 4-t-butylphenyl group, naphthyl group, furyl group, thiophenyl group, pyrroyl group, pyridyl group, furazanyl group, oxazoyl group, imidazoyl group, pyrazolyl group, pyrazyl group, pyrimidyl group, pyridazyl group, benzoimidazoyl group, triazinyl group and the like.

The aromatic groups are preferably aromatic groups having 2 to 30 carbon atoms, more preferably aromatic groups having 2 to 16 carbon atoms, and further preferably aromatic groups having 2 to 10 carbon atoms.

Further, the alkyl groups or the aromatic groups may have a substituent such as hydoxy group, mercapto group, carboxylic group, sulfonic group, phosphonic group, nitro group and halogeno group.

In addition, the ring-opening polymerizable ring is a ring that gives an active species through any one of $\alpha$ ring-

opening, β ring-opening and γ ring-opening generally by a cationic initiator or an anionic initiator.

These are rings described in "Polymer synthesis" (written by Furukawa Junji, p. 99 to 103, issued on 20 February, 1987 by Kagaku Dozin Co., Ltd.), such as cyclopropane ring, cyclopropene ring, cyclopropanone ring, cyclopropenone ring, cyclobutene ring, ethylene oxide ring (1,2-epoxy ring), oxetane ring, tetrahydrofuran ring, ethylene sulfide ring, thiethane ring, ethyleneimine ring, trimethyleneimine ring, pyrrolidine ring, piperidine ring, lactone ring and lactam ring.

Further, these rings may have a substituent, and as the substituent, there can be included one that is similar to one described as the substituent in the group having polymerizable reactive multiple bonds.

Preferable ring-opening polymerizable rings are epoxide ring, oxetane ring, or thiethane ring, and these having alkylene groups as a linking group are further preferable, specifically, there are included epoxy alkylene groups, oxacyclobutyl alkylene groups and thiranyl alkylene groups. In particular, glycidyl group and oxacyclobutyl methylene group are preferable.

As described above, though the ligand L has at least one group having a polymerizable reactive multiple bonds or a group having a ring-opening polymerizable ring, when there is a plurality, they may be the same or different, needless to say, may have a group having polymerizable reactive multiple bonds and also a group having a ring-opening polymerizable ring.

Among them, the ligand L of the present invention preferably has a group having polymerizable reactive multiple bonds.

As described in the requirement (ii), the ligand L of the present invention has 5 of more coordination atoms in a molecule. Here, a coordination atom represents an atom that has an unshared electron pair giving electrons in a vacant orbital of a metal atom and can form a coordinate bond with the metal atom, as described in "Iwanami, Physical and chemical science dictionary, fourth edition" (edited by Kubo Ryougo, et. al, issued on 10 January, 1991, p. 966, by Iwanami Shoten Publisher).

The number of coordination atoms present in the ligand L is preferably 5 or more and 20 or less, more preferably 5 or more and 12 or less, and further preferably 7 or more and 10 or less.

Further, for metal atoms in the multinuclear complex of the present invention, the number of coordinate bond with the ligand L is each preferably 3 or more, more preferably 3 or more, and 20 or less, further preferably 3 or more, and 7 or less, more further preferably 4 or more, and 6 or less, and particularly preferably 4 or 5.

The coordination atom is preferably an atom selected from the group consisting of a carbon atom, a nitrogen atom, an oxygen atom, a phosphorus atom and a sulfur atom, and more preferably a nitrogen atom, an oxygen atom, a phosphorus atom or a sulfur atom, above all, a nitrogen atom, an oxygen atom or a sulfur atom is preferable, a nitrogen atom or an oxygen atom is particularly preferable. Additionally, a plurality of coordination atoms may be the same or different each other.

As shown in the requirement (iii), the ligand L itself, namely, a compound which can be a ligand L is soluble in a solvent. According to the above, it is preferable because production of the multinuclear complex itself of the present invention becomes easy. In addition the solvent is not particularly limited, and a solvent which gives a complex reaction smoothly to obtain a multinuclear complex easily is preferable.

Further, for coordination atoms in the ligand L of the present invention, a part or whole thereof is preferably a nitrogen atom having a double bond with carbon. Containing such nitrogen atom as a coordination atom is preferable because redox catalyst activity, particularly, catalyst activity in peroxide decomposition reaction is superior.

Herein, a nitrogen atom having a double bond with carbon, for example, includes such as a nitrogen atom of an imino group obtained by condensation of a carbonyl group of a ketone compound or an aldehyde compound with an amine compound, and a nitrogen atom of an aromatic heterocyclic ring having a carbon-nitrogen double bond.

Having the aromatic heterocyclic ring having a carbon-nitrogen double bond in a ligand L means that a monovalent or multivalent aromatic heterocyclic group obtained by removing one or more hydrogen atoms from an aromatic heterocyclic molecule or a condensed ring molecule containing these aromatic heterocyclic molecule are present in the ligand L.

The aromatic heterocyclic group may have a substituent.

As the aromatic heterocyclic molecule, an aromatic heterocyclic molecule such as imidazole, pyrazole, 2H-1,2,3-triazole, 1H-1,2,4-triazole, 4H-1,2,4-triazole, 1H-tetrazole, oxazole, isooxazole, thiazole, isothiazole, furazan, pyridine, pyrazine, pyrimidine, pyridazine, 1,3,5-triazine, 1,3,4,5-tetrazine is exemplified.

Further, as the condensed ring molecule containing the aromatic heterocyclic molecules, benzoimidazole, 1H-indazole, benzooxazole, benzothiazole, quinoline, isoquinoline, cinnoline, quinazoline, quinoxaline, phthalazine, 1,8-naphthylidine, pteridine, phenanthridine, 1,10-phenanthroline, purine, perimidine and the like are exemplified.

Herein, a condensed ring represents a ring structure that each ring shares 2 or more atoms in a ring compound having 2 or more rings, as described in "Comprehensive dictionary on Chemistry" (first edition, 1994, Tokyo Kagaku Dozin Co., Ltd.).

Among the aromatic heterocyclic groups exemplified, preferable is a monovalent or multivalent aromatic heterocyclic group obtained by removing one or more hydrogen atoms from an aromatic heterocyclic molecule or the

condensed ring molecule, such as imidazole, pyrazole, pyridine, pyrazine, pyrimidine, benzoimidazole, 1H-indazole, quinoline, isoquinoline, cinnoline, phthalazine, 1,8-naphthylidine and purine.

Herein, in the case where there is a substituent in the aromatic heterocyclic group, those that are similar to the exemplification of the group having polymeriazable reactive multiple bonds can be included. The position of a substituent is arbitrary, the number of substituents and the combinations thereof are arbitrary.

Among them, an aromatic heterocyclic group is preferable as the group containing a carbon-nitrogen double bond.

The multinuclear complex of the present invention may have other ligand in addition to the ligand L. Other ligand may be an ionic or an electrically neutral compound, and when it has a plurality of such other ligands, these other ligands may be the same or different.

As an electrically neutral compound in the other ligand, there are exemplified: a nitrogen atom-containing compound such as ammonia, pyridine, pyrrole, pyridazine, pyrimidine, pyrazine, 1,2,4-triazine, pyrazole, imidazole, 1,2,3-triazole, oxazole, isooxazole, 1,3,4-oxadiazole, thiazole, isothiazole, indole, indazole, quinoline, isoquinoline, phenanthridine, cinnoline, phthalazine, quinazoline, quinoxaline, 1,8-naphthylidine, acridine, 2,2'-bipyridine, 4,4'-bipyridine, 1,10-phenanthroline, ethylenediamine, propylenediamine, phenylenediamine, cyclohexanediamine, pyridine N-oxide, 2,2'-bipyridine N,N'-dioxide, oxamide, dimethyl glyoxime and o-aminophenol; an oxygen atom-containing compound such as water, phenol, oxalic acid, catechol, salicylic acid, phthalic acid, 2,4-pentanedione, 1,1,1-trifluoro-2,4-pentanedione, hexafluoropentanedione, 1,3-diphenyl-1,3-propanedione, 2,2'-binaphthol; a sulfur-containing compound such as dimethyl sulfoxide and urea; and a phosphorous-containing compound such as 1,2-bis(dimethylphosphino)ethane and 1,2-phenylenebis(dimethylphosphine).

Preferable are ammonia, pyridine, pyrrole, pyridazine, pyrimidine, pyrazine, 1,2,4-triazine, pyrazole, imidazole, 1,2,3-triazole, oxazole, isooxazole, 1,3,4-oxadiazole, indole, indazole, quinoline, isoquinoline, phenanthridine, cinnoline, phthalazine, quinazoline, quinoxaline, 1,8-naphthylidine, acridine, 2,2'-bipyridine, 4,4'-bipyridine, 1,10-phenanthroline, ethylenediamine, propylenediamine, phenylenediamine, cyclohexanediamine, pyridine N-oxide, 2,2'-bipyridine N,N'-dioxide, oxamide, dimethyl glyoxime, o-aminophenol, water, phenol, oxalic acid, catechol, salicylic acid, phthalic acid, 2,4-pentanedione, 1,1,1-trifluoro-2,4-pentanedione, hexafluoropentanedione, 1,3-diphenyl-1,3-propanedione and 2,2'-binaphthol;

More preferable are ammonia, pyridine, pyrrole, pyridazine, pyrimidine, pyrazine, 1,2,4-triazine, pyrazole, imidazole, 1,2,3-triazole, oxazole, isooxazole, 1,3,4-oxadiazole, indole, indazole, quinoline, isoquinoline, phenanthridine, cinnoline, phthalazine, quinazoline, quinoxaline, 1,8-naphthylidine, acridine, 2,2'-bipyridine, 4,4'-bipyridine, 1,10-phenanthroline, ethylenediamine, propylenediamine, phenylenediamine, cyclohexanediamine, pyridine N-oxide, 2,2'-bipyridine N,N'-dioxide, o-aminophenol, phenol, catechol, salicylic acid, phthalic acid, 1,3-diphenyl-1,3-propanedione, and 2,2'-binaphthol.

Among them, further preferable are pyridine, pyrrole, pyridazine, pyrimidine, pyrazine, pyrazole, imidazole, oxazole, indole, quinoline, isoquinoline, acridine, 2,2'-bipyridine, 4,4'-bipyridine, 1,10-phenanthroline, phenylenediamine, pyridine N-oxide, 2,2'-bipyridine N,N'-dioxide, o-aminophenol and phenol.

Further, as an anionic ligand, there are included hydroxide ion, peroxide, super oxide, cyanide ion, thiocyanate ion, halide ions such as fluoride ion, chloride ion, bromide ion and iodide ion; sulfate ion, nitrate ion, carbonate ion, perchlorate ion, tetrafluoroborate ion, tetraarylborate ions such as tetraphenylborate ion, hexafluorophosphate ion, sulfonate ions such as methanesulfonate ion, trifluoromethanesulfonate ion, p-toluenesulfonate ion, benzenesulfonate ion and dodecylbenzenesulfonate ion, dodecylsulfate ion, phosphate ion, phosphite ion, phenylphosphonate ion, diphenylphosphonate ion, acetate ion, trifluoroacetate ion, propionate ion, benzoate ion, hydroxy ion, metal oxide ions, methoxide ion, ethoxide ion, vinylbenzoate ion, acrylate ion, methacrylate ion and the like.

Preferably, there are exemplified hydroxide ion, sulfate ion, nitrate ion, carbonate ion, perchlorate ion, tetrafluoroborate ion, tetraphenylborate ion, hexafluorophosphate ion, methanesulfonate ion, trifluoromethanesulfonate ion, p-toluenesulfonate ion, benzenesulfonate ion and dodecylbenzenesulfonate ion, phosphate ion, phosphite ion, phenylphosphonate ion, diphenylphosphonate ion, acetate ion, trifluoroacetate ion, vinylbenzoate ion, acrylate ion and methacrylate ion.

Among them, preferable are hydroxide ion, sulfate ion, nitrate ion, carbonate ion, tetraphenylborate ion, trifluoromethanesulfonate ion, p-toluenesulfonate ion, acetate ion, trifluoroacetate ion, vinylbenzoate ion, acrylate ion and methacrylate ion.

Further, the ions exemplified as the anionic ligand may act as a counter ion to electrically neutralize the multinuclear metal complex itself of the present invention.

In addition, there is a case that the multinuclear complex of the present invention has a cationic counter ion for maintaining electrical neutrality. As the cationic counter ion, alkali metal ions, alkaline earth metal ions, tetraalkylammonium ions such as tetra (n-butyl) ammonium ion and tetraethylammonium ion, and tetraarylphosphonium ions such as tetraphenylammonium ion are exemplified, specifically, there are included lithium ion, sodium ion, potassium ion, rubidium ion, cesium ion, magnesium ion, calcium ion, strontium ion, barium ion, tetra(n-butyl)ammonium ion, tetraethylammonium

ion and tetraphenylphosphonium ion; and more preferably tetra(n-butyl)ammonium ion, tetraethylammonium ion and tetraphenylphosphonium ion,

Among them, tetra(n-butyl)ammonium ion and tetraethylammonium ion are preferable as a cationic counter ion.

Additionally, using various counter ions suitably can also adjust such as solubility or dispersibility of a multinuclear complex in a solvent.

The multinuclear complex of the present invention has a plurality of metal atoms, and one or more of the ligands L described above, in particular, it is preferable that at least 2 metal atoms of a plurality of metal atoms are located closely in a molecule.

In this case, as an index that metal atoms are located closely, when the two metal atoms are denoted as $M^1$, $M^2$, and coordination atoms bonding to $M^1$ and $M^2$ are denoted as $AM^1$ and $AM^2$, respectively, they preferably have a combination of $AM^1$ and $AM^2$ such that the minimum of a covalent bond linking $AM^1$ and $AM^2$ is 4 or less. The minimum is more preferably 3 or less, further preferably 2 or less, and particularly preferably 1.

Particularly preferably, it is a multinuclear complex that two metal atoms ($M^1$, $M^2$) selected from the plurality of metal atoms form a coordinate bond with the same coordination atom. This means that $M^1$ and $M^2$ are crosslinked coordinatively with the same coordination atom. According to the above, the distance between $M^1$ and $M^2$ becomes small, and an interaction of between two metal atoms tends to be exhibited, and thus a catalyst activity of the multinuclear complex becomes higher.

The $M^1$ and $M^2$ may also both be coordination atoms present in the ligand L, or may both be coordination atoms present in a ligand other than the ligand L.

In the multinuclear complex, coordination atoms crosslink-bonding to two metal atoms may also be coordination atoms of the ligand L or coordination atoms in a ligand other than the ligand L.

In addition, the multinuclear complex of the present invention preferably has a molecular weight of 6000 or less. It is preferable because synthesis of a multinuclear complex itself is easy if it is within such molecular weight range. The molecular weight is more preferably 5000 or less, further preferably 4000 or less, and particularly preferably 2000 or less.

Further, the lower molecular weight of a multinuclear complex is preferable because operations become simple in polymerization or copolymerization of the multinuclear complex described later.

Next, preferable compounds as a ligand L related to the multinuclear complex of the present invention will be explained. The ligand L, as previously described, preferably contains a nitrogen atom on a carbon-nitrogen double bond as a coordination atom, and in particular, more preferably has a nitrogen atom on the carbon-nitrogen double bond in an aromatic heterocyclic group.

In this way, the ligand L having a nitrogen atom on a carbon-nitrogen double bond as a coordination atom can be exemplified by a ligand that a hydrogen atom in the compound described in a document (Anna L. Gavrilova and Brice Bosnich, Chem. Rev. 2004, 104, 349.), that is, Ligand Numbers 52 to 55, 56a, 56b, 56c, 57a, 57b, 57c, 57d, 58a, 58b, 58c and 60 in Table 5 (p. 357) ; Ligand Numbers 73 and 74 in Table 7 (p. 360) ; Ligand Numbers 79, 80, 83 and 85 in Table 8 (p. 362); Ligand Numbers 90, 91 and 92 in Table 9 (p. 364); Ligand Numbers 100 to 103, 105 to 108, 110, 111, and 113 to 118 in Table 10 (p. 366); Ligand Numbers 123 to 126, 129, 131, 132, 134 to 138, and 141 to 147 in Table 11 (p. 370 to 371); Ligand Numbers 151, 152, and 154 to 157 in Table 12 (p. 373); Ligand Numbers 166 and 167 in Table 13 (p. 376) ; Ligand Number 174 in Table 14 (p. 377); and Ligand Numbers 177 and 179 in Table 15 (p. 378) described in (Anna L. Gavrilova and Brice Bosnich, Chem. Rev. 2004, 104, 349.) was substituted with the group having polymerizable reactive multiple bonds and/or a group containing a ring-opening polymerizable ring.

Among the above exemplifications, as a particularly preferable ligand, one having an aromatic heterocyclic group containing a carbon-nitrogen double bond is preferable, which can be exemplified by a ligand that a hydrogen atom in the compound expressed by Ligand Numbers 52 to 55, 56a, 56b, 56c, 57a, 57b, 57c, 57d, 58a, 58b, 58c and 60 in Table 5 (p. 357); Ligand Numbers 73 and74 in Table 7 (p. 360) ; Ligand Numbers 79, 80, 83 and 85 in Table 8 (p. 362); Ligand Numbers 90, 91 and 92 in Table 9 (p. 364); Ligand Numbers 100, 101, 106 to 108, 110, 111 and 113 to 118 in Table 10 (p. 366); Ligand Numbers 123, 124, 126, 129, 131, 132, 134 to 138 and 141 to 147 in Table 11 (p. 370 to 371); Ligand Numbers 155 to 157 in Table 12 (p. 373); Ligand Number 174 in Table 14 (p. 377); and Ligand Numbers 177 and 179 in Table 15 (p. 378) was substituted with the group having polymerizable reactive multiple bonds and/or a group containing a ring-opening polymerizable ring.

The ligand L related to the multinuclear complex of the present invention preferably has the aromatic heterocyclic group described above and a molecular weight of 6000 or less, and from such viewpoints, in particular, a compound expressed by the following formula (1) is preferable:

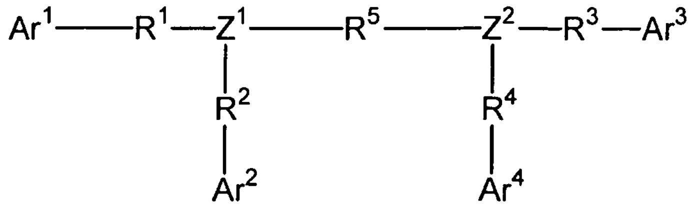

(1)

wherein $Ar^1$, $Ar^2$, $Ar^3$ and $Ar^4$ (hereinafter sometimes denoted as $Ar^1$ to $Ar^4$) each independently represents an aromatic heterocyclic group, $R^1$, $R^2$, $R^3$, $R^4$ and $R^5$ (hereinafter sometimes denoted as $R^1$ to $R^5$) represent a divalent linking group, $Z^1$ and $Z^2$ each independently represents a nitrogen atom or a trivalent group; at least one of $Ar^1$ to $Ar^4$ and $R^1$ to $R^5$ has a group having polymerizable reactive multiple bonds, and/or a ring-opening polymerizable ring.

Herein, $Ar^1$ to $Ar^4$ are preferably the aromatic heterocyclic groups exemplified above, for example, there can be exemplified imidazolyl group, pyrazolyl group, 2H-1,2,3-triazolyl group, 1H-1,2,4-triazolyl group, 4H-1,2,4-triazolyl group, 1H-tetrazolyl group, oxazolyl group, isooxazolyl group, thiazolyl group, isothiazolyl group, furazyl group, pyridyl group, pyradyl group, pyrimidyl group, pyridazyl group, 1,3,5-triazyl group and 1,3,4,5-tetrazyl group.

As an aromatic heterocyclic ring, they may also be a condensed ring group thereof for example, there can be exemplified benzoimidazoyl group, 1H-imidazoyl group, benzooxazoyl group, benzothiazoyl group, quinolyl group, isoquinolyl grouop, cinnolyl group, quinazoyl group, quinoxalyl group, phtahlazyl group, 1,8-naphthylidyl group, pteridyl group, carbazolyl group, phenanthridyl group, 1,10-phenanthrolyl group, puryl group, and perimidyl group.

In addition, these aromatic heterocyclic rings may have a substituent. As an example of the substituent, it is similar to the substituent exemplified in the group having a multiple bond. Further, the substituting position and the number of the substituent, and the combinations thereof are arbitrary. The group having polymerizable reactive multiple bonds or the group having a polymerizable ring may be bonded to the aromatic heterocyclic group.

As the aromatic heterocyclic rings $Ar^1$ to $Ar^4$ in the formula (1), preferably, they are benzoimidazoyl group, pyridyl group, imidazoyl group, pyrazoyl group, oxazoyl group, thiazolyl group, isooxazolyl group, isothiazolyl group, pyrazyl group, pyrimidiyl group, pyridazyl group, and N-alkylbenzoimidazoyl groups and N-alkylimidazoyl groups having the alkyl group exemplified above on its nitrogen;

More preferable are benzoimidazoyl group, pyridyl group, imidazoyl group, pyrazoyl group, pyrazyl group, pyrimidiyl group, pyridazyl group, N-alkylbenzoimidazoyl groups and N-alkylimidazoyl groups;

Further preferable are benzoimidazoyl group, N-alkylbenzoimidazoyl groups, pyridyl group, imidazoyl group, N-alkylimidazoyl groups and pyrazoyl group; and

Particularly preferable are pyridyl group, N-alkylbenzoimidazoyl groups and N-alkylimidazoyl groups.

$R^5$ is also a divalent group which may have a coordination atom or a group containing a coordination atom, it is selected from an alkylene group, a divalent aromatic group and a divalent organic group containing a hetero atom shown below, and may be a group that these are arbitrarily bonded and combined.

As an example of the alkylene group, there are included alkylene groups that are obtained by removing two hydrogen atoms from a saturated hydrocarbon having about 1 to 50 total carbon atoms such as methane, ethane, propane, butane, octane, decane, icosane, triacontane, pentacontane, cycloheptane and cyclohexane.

These alkylene groups may also have substituents in any position, the number of the substituents and the combinations thereof are arbitrary, and as the substituents, the ones similar to the exemplifications in the group having polymerizable reactive multiple bonds can be included.

Herein, as the alkylene group, the carbon atoms contained are preferably 1 to 30, more preferably 1 to 16, further preferably 1 to 8, and particularly preferably 1 to 4.

As an example of the divalent aromatic group, there are included groups that are obtained by removing two hydrogen atoms from an aromatic compound, a heterocyclic compound or a compound having substituents in these compounds such as benzene, naphthalene, anthracene, tetracene, biphenyl, acenaphthylene, phenalene, pyrene, furan, thiophene, pyrrole, pyridine, oxazole, isooxazole, thiazole, isothiazole, imidazole, pyrazole, pyrazine, pyrimidine, pyridazine, benzofuran, isobenzofuran, 1-benzothiophene, 2-benzothiophene, indole, isoindole, indolizine, carbazole, xanthene, quinoline, isoquinoline, 4H-quinolizine, phenanthridine, acrydine, 1,8-naphthyridine, benzoimidazole, 1H-indazole, quinoxaline, quinazoline, cinnoline, phthalazine, purine, pteridine, perimidine, 1,10-phenanthroline, thianthorene, phenoxathiin, phenoxadine, phenothiazine, phenazine and phenarsazine.

Among them, preferable are groups that are obtained by removing two hydrogen atoms from a compound selected from benzene, phenol, p-cresol, naphthalene, biphenyl, furan, thiophene, pyrrole, pyridine, oxazole, isooxazole, thiazole, isothiazole, imidazole, pyrazole, pyrazine, pyrimidine, pyridazine, benzofuran, isobenzofuran, 1-benzothiophene, 2-benzothiophene, indole, isoindole, indolizine, carbazole, xanthene, quinoline, isoquinoline, 1,8-naphthyridine, benzoimidazole, 1H-indazole, quinoxaline, quinazoline, cinnoline, phthalazine, purine, pteridine and perimidine;

More preferable are groups that are obtained by removing two hydrogen atoms from a compound selected from benzene, naphthalene, biphenyl, pyrrole, pyridine, oxazole, isooxazole, thiazole, isothiazole, imidazole, pyrazole, pyrazine, pyrimidine, pyridazine, indole, isoindole, quinoline, isoquinoline, 1,8-naphthyridine, benzoimidazole, 1H-indazole, quinoxaline, quinazoline, cinnoline and phthalazine

Further preferable are groups that are obtained by removing two hydrogen atoms from a compound selected from benzene, phenol, p-cresol, naphthalene, biphenyl, pyrrole, pyridine, imidazole, pyrazole, pyrazine, pyridazine, indole, isoindole, quinoline, isoquinoline, 1,8-naphthyridine, benzoimidazole, 1H-indazole, quinoxaline, quinazoline, cinnoline and phthalazine; and

Particularly preferable are groups that are obtained by removing two hydrogen atoms from a compound selected from phenol, p-cresol, pyridine, pyrazole, pyridazine, 1,8-naphthyridine, 1H-indazole and phthalazine.

Substituents in these divalent aromatic groups may be located at an arbitrary position, the number and the combinations thereof are arbitrary. As the substituents, the substituents exemplified in the group having polymerizable reactive multiple bonds can be included. Further, as the substituent, it may have the group having polymerizable reactive multiple bonds and/or the group having a ring-opening polymerizable ring of the present invention.

As the divalent organic group containing hetero atoms, for example, groups expressed by the following (E-1) to (E-10) are included.

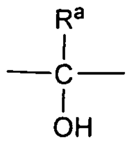
(E-1)

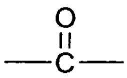
(E-2)

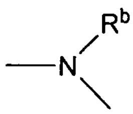
(E-3)

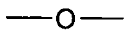
(E-4)

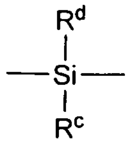
(E-5)

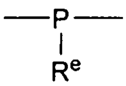
(E-6)

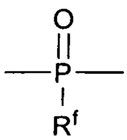
(E-7)

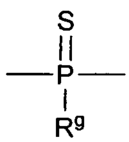
(E-8)

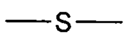
(E-9)

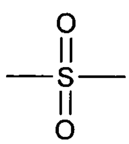
(E-10)

wherein $R^a$, $R^e$, $R^f$ and $R^g$ represent an alkyl group having 1 to 50 carbon atoms, an aromatic group having 2 to 60 carbon atoms, an alkoxy group having 1 to 50 carbon atoms, an aryloxy group having 2 to 60 carbon atoms, a hydroxyl group, or a hydrogen atom. $R^b$ represents an alkyl group having 1 to 50 carbon atoms, an aromatic group having 2 to 60 carbon atoms, or a hydrogen atom, $R^d$ and $R^c$ represent an alkyl group having 1 to 50 carbon atoms, or an aromatic group having 2 to 60 carbon atoms.

As a bifunctional hetero atom functional group of $R^5$, (E-1), (E-2), (E-3), (E-4), (E-5), (E-7), (E-8) and (E-10) are preferable, (E-1), (E-2), (E-4), (E-7) and (E-10) are more preferable, and (E-1) and (E-7) are further preferable.

In particular, $R^5$ preferably contains a functional group capable of coordinating to a metal atom. As the functional group capable of coordinating to a metal atom, there are included hydroxyl group, carboxyl group, mercapto group, sulfonic acid group, phosphonic acid group, nitro group, cyano group, ether group, acyl groups, ester groups, amino group, carbamoyl group, acid amide group, phospholyl group, thiophospholyl group, sulfide group, sulfonyl group, pyrrolyl group, pyridyl group, oxazolyl group, isooxazolyl group, thiazolyl group, isothizoyl group, imidazolyl group, pyrazolyl group, pyrazyl group, pyrimidyl group, pyridazyl group, indolyl group, isoindolyl group, carbazolyl group, quinolyl group, isoqunolyl group, 1,8-naphthyridyl group, benzoimidazolyl group, 1H-indazolyl group, quinoxalyl group, quinazolyl group, cinnolyl group, phtalazyl group, puryl group, pteridyl group and permidyl group.

Preferably, there are included hydroxyl group, carboxyl group, sulfonic acid group, phosphonic acid group, nitro group, cyano group, ether group, acyl groups, amino group, phospholyl group, thiophospholyl group, sulfonyl group, pyrrolyl group, pyridyl group, oxazolyl group, isooxazolyl group, thiazolyl group, isothizoyl group, imidazolyl group, pyrazolyl group, pyrazyl group, pyrimidyl group, pyridazyl group, indolyl group, isoindolyl group, quinolyl group, isoqunolyl group, 1,8-naphthyridyl group, benzoimidazolyl group, 1H-indazolyl group, quinoxalyl group, quinazolyl group, cinnolyl group, phtalazyl group, puryl group, pteridyl group and permidyl group; more preferably, hydroxyl group, carboxyl group, sulfonic acid group, a phosphonic acid group, cyano group, ether group, acyl groups, amino group, phospholyl group, sulfonyl group, pyridyl group, imidazolyl group, pyrazolyl group, pyrimidyl group, pyridazyl group, quinolyl group, isoqunolyl group, 1,8-naphthyridyl group, benzoimidazolyl group, 1H-indazolyl group, cinnolyl group, phtalazyl group and

pteridyl group.

As a particularly preferable $R^5$, the following (R5-1), (R5-2), (R5-3) or (R5-4) can be exemplified, and (R5-1) is particularly preferable.

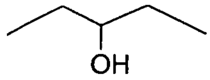

(R5-1)

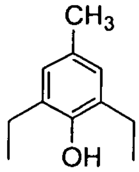

(R5-2)

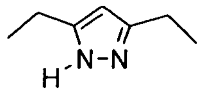

(R5-3)

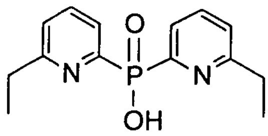

(R5-4)

Herein, a hydroxyl group in (R5-1) and (R5-2), a pyrazole ring in (R5-3), and a phosphinic group in (R5-4) sometimes become anionic by releasing a proton in coordinating to a metal atom as a ligand.

In the formula (3), $R^1$ to $R^4$ are a divalent group that may be substituted, and may be each independently the same or different. As an example of $R^1$ to $R^4$, the one similar to the alkylene group, a divalent aromatic group and the divalent organic group containing a hetero atom, and the divalent group that these groups are bonded and combined arbitrarily exemplified in $R^5$ can be included.

As an example of $R^1$ to $R^4$, a methylene group, a 1,1-ethylene group, a 2,2-propylne group, a 1,2-ethylene group and a 1,2-phenylene group are preferable, and a methylene group and a 1,2-ethylene group are more preferable.

$Z^1$ and $Z^2$ in the formula (1) are selected from a nitrogen atom or a trivalent organic group, and as the trivalent organic group, the following groups are included.

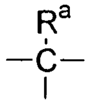

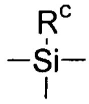

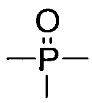

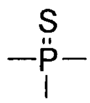

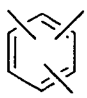

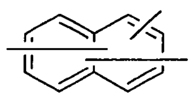

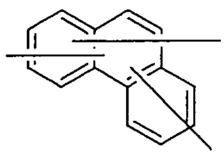

wherein $R^a$ and $R^c$ are the same meanings as described above.

Above all, it is preferable that either $Z^1$ or $Z^2$ is a nitrogen atom, and particularly preferable that both are nitrogen atoms. Specifically, a compound expressed by the foregoing formula (1) is preferably a compound expressed by the formula (2).

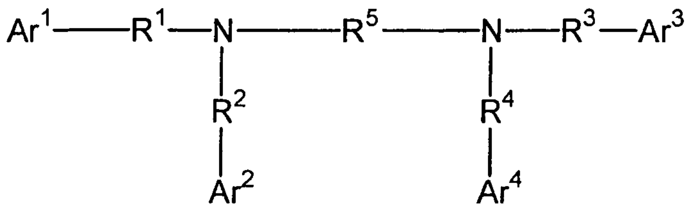

(2)

wherein $Ar^1$ to $Ar^2$, and $R^1$ to $R^5$ are the same definitions as in the formula (1), at least one of them has a group having polymeriazable reactive multiple bonds or a group having a polymerizable ring.

Among the compounds expressed by the formula (2), a compound expressed by the following formula (3a) or (4a) is further preferable.

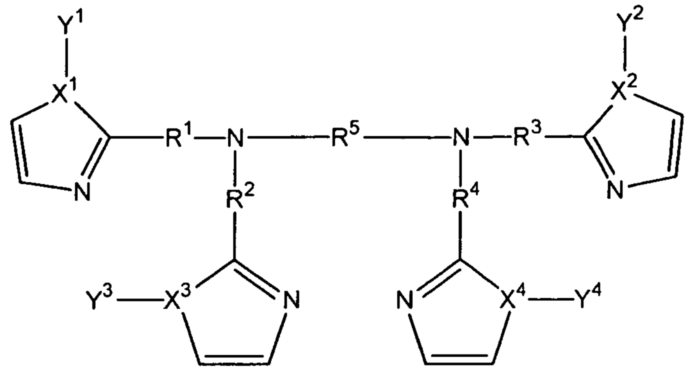

(3a)

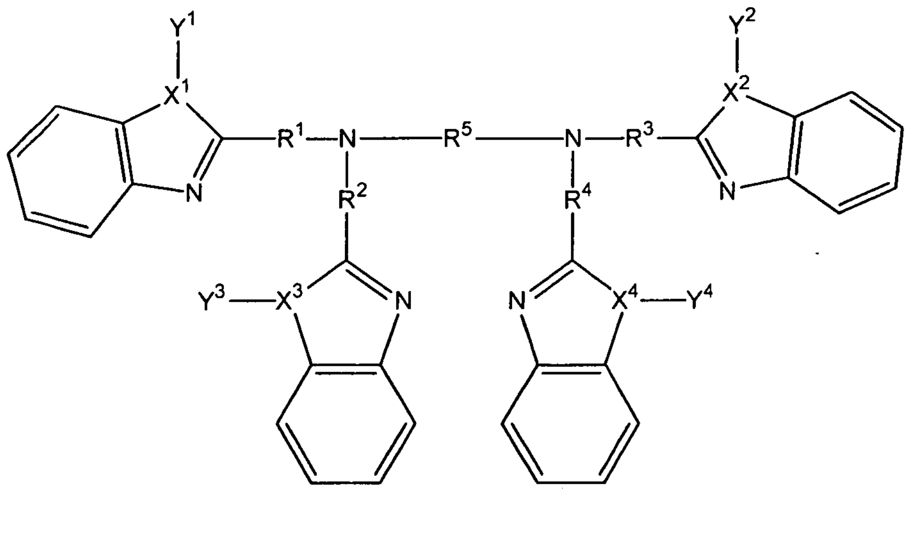

(4a)

In the formulas (3a) and (4a), $R^1$ to $R^5$ are the same meanings as in the formula (1). $X^1$, $X^2$, $X^3$ and $X^4$ (hereinafter sometimes denoted as X1 to $X^4$) are selected from a nitrogen atom or CH. $Y^1$, $Y^2$, $Y^3$ and $Y^4$ (hereinafter sometimes denoted as $Y^1$ to $Y^4$) represent a hydrogen atom, an alkyl group having 1 to 50 carbon atoms, an aromatic group having 2 to 60 carbon atoms, a group having a polymerizable reactive carbon-carbon double bond or a group having a ring-opening polymerizable ring, and at least one of $Y^1$ to $Y^4$ is a group having a polymerizable reactive carbon-carbon double bond or a group having a ring-opening polymerizable ring.

Among the compounds expressed by the formula (3a) or (4a), a compound expressed by the following formula (3b) or (4b) is easy for production and particularly preferable.

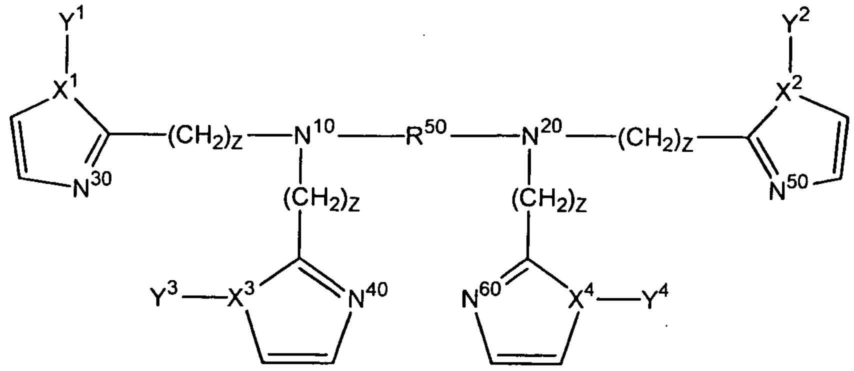

(3b)

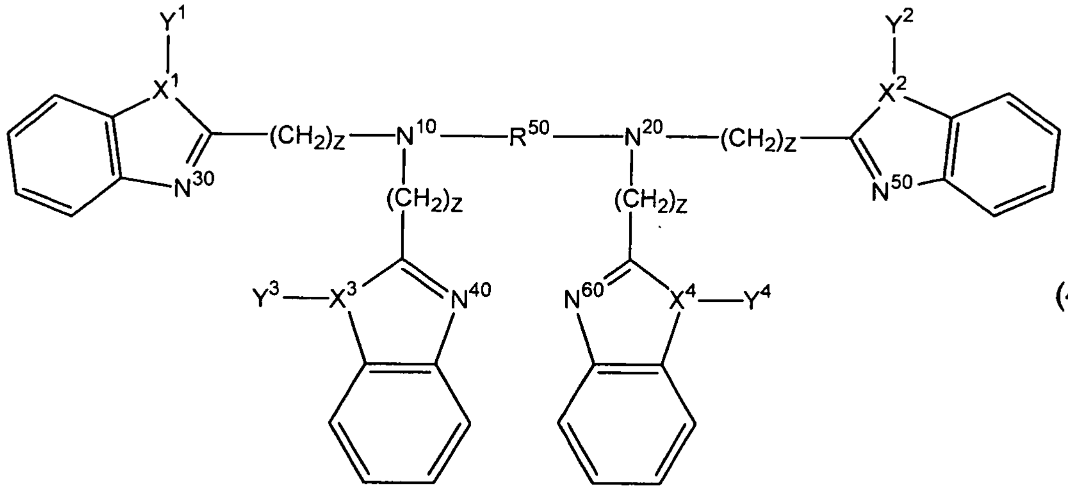

(4b)

In the formulas (3b) and (4b), $X^1$, $X^2$, $X^3$ and $X^4$ (hereinafter sometimes denoted as $X^1$ to $X^4$), and $Y^1$, $Y^2$, $Y^3$ and $Y^4$ (hereinafter sometimes denoted as $Y^1$ to $Y^4$) are the same meanings as in the formula (3a) or (4a). At least one of $Y^1$ to $Y^4$ is a group having a polymerizable reactive carbon-carbon double bond or a group having a ring-opening polymerizable ring. Z represents an integer of 1 or 2. $N^{10}$ and $N^{20}$ represent a nitrogen atom bonding with $R^{50}$, and $N^{30}$, $N^{40}$, $N^{50}$ and $N^{60}$ (hereinafter sometimes denoted as $N^{30}$ to $N^{60}$) represent a nitrogen atom in an aromatic heterocyclic group. $R^{50}$ represents a divalent group having the minimum of a covalent bond linking $N^{10}$ and $N^{20}$ of 2 or more, and 14 or less.

$R^{50}$ represents a divalent group having the minimum of a covalent bond linking $N^{10}$ and $N^{20}$ of 2 or more, and 14 or less. As a specific example, the minimum is 4 in the (R5-1), the minimum is 6 in the (R5-2), the minimum is 6 in the (R5-3), and the minimum is 10 in the (R5-4).

A synthesis method of the compound expressed by the formulas (3b) and (4b) can use various methods. As an example thereof, a synthetic method of (4b) that includes the following formulas (100) and (200) and where $X^1$ to $X^4$ are a nitrogen atom can be included. By such a method, (4b) having $R^{50}$ with various structures of the (R5-1), the (R5-2), the (R5-3) and the (R5-4) can be synthesized.

Reaction of a diamine compound having $CO_2R^{100}$ group and o-phenylenediamine:

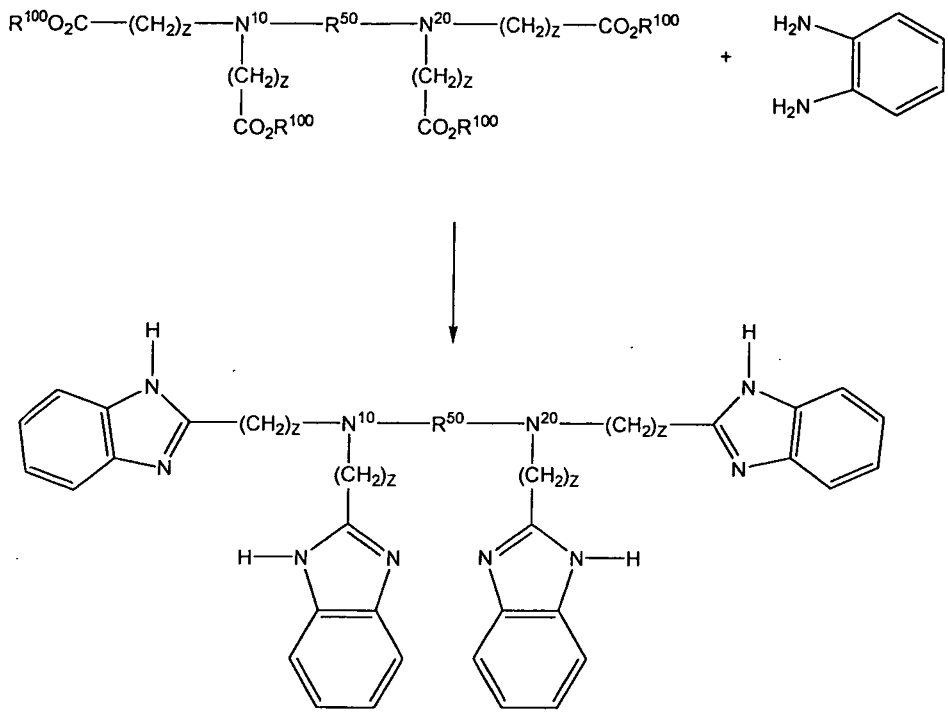

(100)

wherein $R^{100}$ represents either a hydrogen atom, the alkyl group, or the aromatic group. Z, $N^{10}$, $N^{20}$ and $R^{50}$ are the same meanings as in the formula (4).

Substitution reaction of N-H bond on a benzoimidazolyl group:

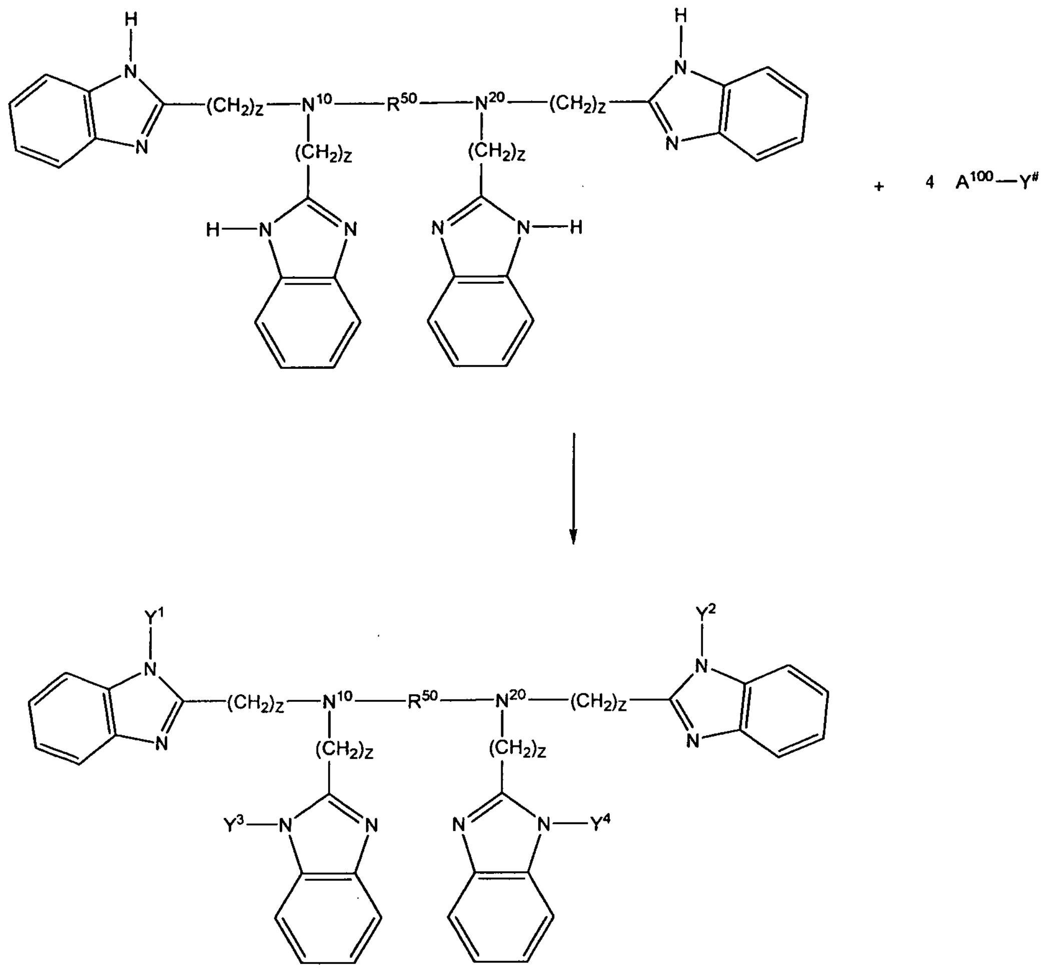

(200)

In the figure, $A^{100}$ represents any of a chlorine atom, a bromine atom, an iodine atom, a p-toluenesulfonyloxy group, a methylsulfonyloxy group, or a trifluoromethylsulfonyloxy group, which may be the same or different each other. Z, $N^{10}$, $N^{20}$ and $R^{50}$ are the same meanings as in the formula (100). $Y^{\#}$ represents any one of $Y^1$ to $Y^4$ in the formula (4b), the same meanings as in the formula (4b).

In the compound expressed by the formula (3b) or (4b), Z is preferably 1, specifically, a compound expressed by the following (3c) or (4c) is preferable.

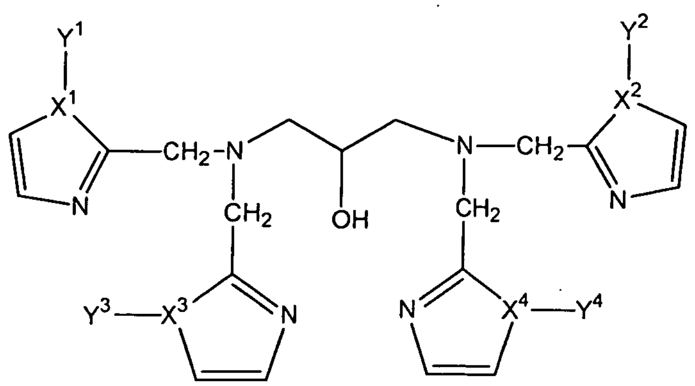

(3c)

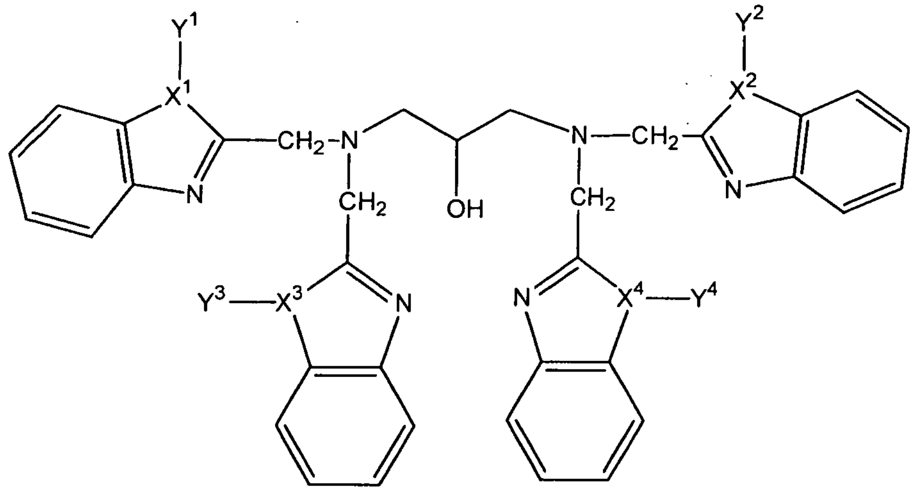

(4c)

In the formulas (3c) and (4c), $X^1$ to $X^4$, and $Y^1$ to $Y^4$ are the same meanings as in the formula (3a) or (4a), and at least one of $Y^1$ to $Y^4$ is a group having a polymerizable reactive carbon-carbon double bond or a group having a ring-opening polymerizable ring.

In the compound expressed by the formula (3b), (4b), (3c) or (4c), at least one of $Y^1$ to $Y^4$ is a group having a polymerizable reactive carbon-carbon double bond or a group having a ring-opening polymerizable ring, and a preferable group, as described above, is a group selected from vinyl group, styryl group, vinylbenzyl group, allyl group, glycidyl group, and oxacyclobutylmethylene group.

Herein, it is preferable that two or more of $Y^1$ to $Y^4$ are groups having a polymerizable reactive carbon-carbon double bond or groups having a ring-opening polymerizable ring, it is further preferable that two or more of $Y^1$ to $Y^4$ are groups having a polymerizable reactive carbon-carbon double bond, and it is particularly preferable that all of $Y^1$ to $Y^4$ are groups having a polymerizable reactive carbon-carbon double bond.

Based on the document (Anna L. Gavrilova and Brice Bosnich, Chem. Rev. 2004, 104, 349. in Table 10, Ligands Number 110 (p. 366), as a preferable multinuclear complex, for example, a complex expressed by formula (5) can be exemplified.

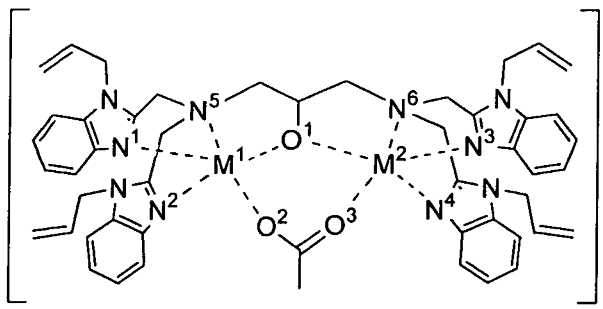

$(CF_3SO_3)_2$ (5)

Herein, in a ligand L, four benzoimidazolyl groups are present as an aromatic heterocyclic group containing a coordination atom ($Ar^1$ to $Ar^4$), in the benzoimidazolyl group, one nitrogen atom as a coordination atom (denoted as $N^1$, $N^2$, $N^3$ and $N^4$) bonds to $M^1$ or $M^2$ (dotted line bonding $M^1$ or $M^2$ represents a coordinate bond), and other nitrogen atoms of this benzoimidazolyl group have a polymerizable reactive allyl group. The ligand L has a methylene group as a group represented by $R^1$ to $R^4$, and a propylene group having an alkolate group being a crosslinking coordination atom (denoted as $O^1$) as $R^5$. Further, a multinuclear complex has an acetate ion (having $O^2$, $O^3$ as coordination atoms) as a ligand other than the ligand L, and two molecules of trifluoromethanesulfonate ions as a counter ion.

Additionally, figures inscribed on a nitrogen coordination atom and an oxygen coordination atom are inscribed for distinction in explaining the number of covalent bonds between coordination atoms described below.

In a complex expressed by the formula (5), the number of covalent bonds present between coordination atoms each bonding to $M^1$ and $M^2$ will be explained.

In the complex in the formula (5);
between $M^1$-$O^1$-$M^2$, $M^1$ and $M^2$ are crosslinked coordinatively with the same coordination atom $O^1$;

between $M^1$-$O^2$-$O^3$-$M^2$, the minimum of the number of covalent bonds linking coordination atoms is 2;
between $M^1$-$O^1$-$N^6$-$M^2$, and between $M^2$-$O^1$-$N^5$-$M^1$, the minimum of the number of covalent bonds linking the coordination atoms is 3; and
between $M^1$-$N^5$-$N^6$-$M^2$, the minimum of the number of covalent bonds linking coordination atoms is 4.

The multinuclear complex having such combinations of coordination atoms is a multinuclear complex having a coordination structure that M1 and M2 are present closely, and such a multinuclear complex is preferable because the catalyst activity is high.

As a production method related to the suitable multinuclear complex, a method that a compound giving a ligand L and a transition metal compound are mixed in a solvent can be included. As the compound giving a ligand L, a precursor compound of the ligand L or a ligand compound, namely, a compound shown by the structure of the ligand itself is included. The transition metal compound is preferably soluble in the solvent. As the preferable ligand L, the one exemplified above is included.

As the preferable transition metal compound, a transition metal salt soluble in a solvent is included. As a preferable transition metal in the transition metal salt, the one exemplified above is included.

Further adding a suitable salt to the complex forming reaction can change counter ions in a complex catalyst into ions derived from the added salt. The preferable added salt contains the preferable counter ions.

As the specific production method, there can be exemplified a synthesis method later described of Mn-bbpr-allyl-OTf, Mn-OAc-(bbpr-$CH_2$St)-OTf, Mn-vb-(bbpr-$CH_2$St)-vb, Mn-vb-(bbpr-$CH_2$St)-DBS, Mn-vb- (bbpr-$CH_2$St) -$HS_{20}$, Co- (bbpr-$CH_2$St)- $BPh_4$, Ni-(bbpr-$CH_2$St)-$BPh_4$, Cu-(bbpr-$CH_2$St)-$BPh_4$ and Fe-(bbpr-$CH_2$St)-$BPh_4$, and synthetic methods shown in the following formulas (010), (020) and (030).

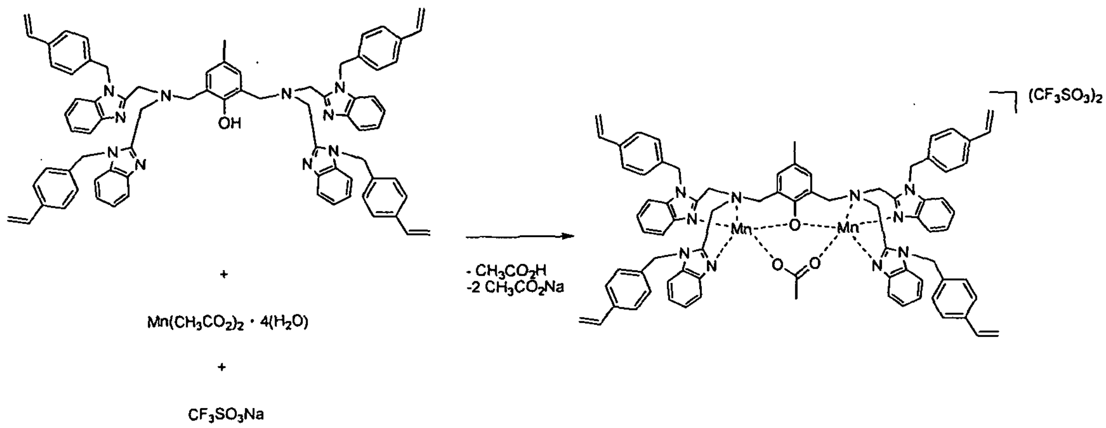


(010)

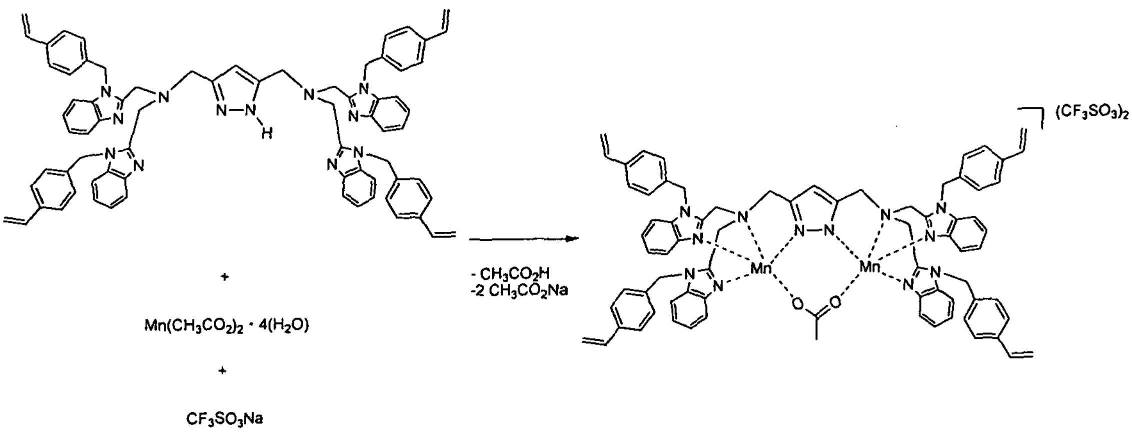


(020)

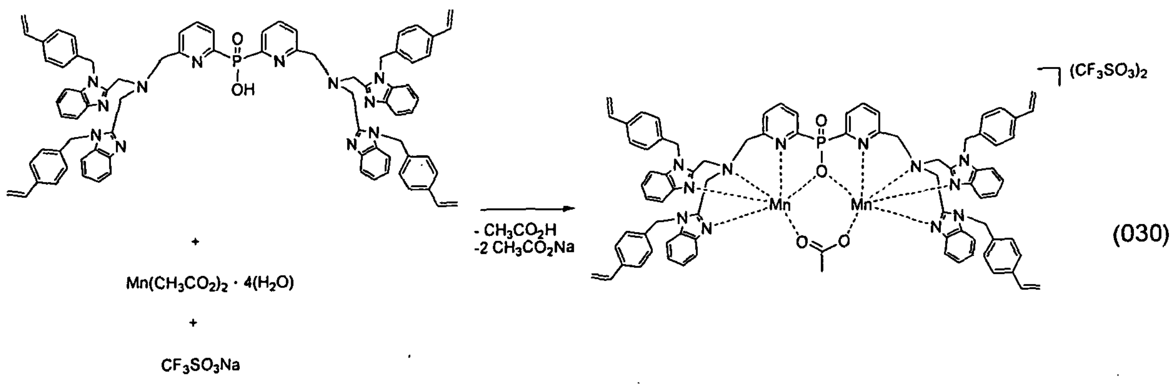


(030)

The multinuclear complex of the present invention can obtain a polymer by a polimerizable reactive group or a ring-opening polymerizable ring of the ligand L, and the polymer can become an excellent catalyst in heat stability.

The multinuclear complex can also be converted into a copolymer by copolymerizing one or a plurality of kinds of polimerizable monomers, and the copolymer can also become an excellent catalyst in heat stability.

As reaction conditions in polymerization or copolymerization, the reaction can be carried out without a solvent, or can be carried out under the presence of a reaction solvent.

When the reaction is carried out without a solvent, there is a case that a reaction substance needs to be ground as a pretreatment. Further, when the copolymerization is carried out without a solvent, a multinuclear complex and a polymerizable monomer must be blended by a technique that a mixture of a multinuclear complex and a polymerizable monomer to be copolymerized are ground and mixed, or a technique of removing a solvent after dissolving and mixing a multinuclear complex and a polymerizable monomer in a reaction solvent, and the like.

Additionally, when the polymerization or the copolymerization is carried out in the presence of a reaction solvent, a multinuclear complex and a polymerizable monomer to be added as needed may be dissolved in the reaction solvent, or being not dissolved therein, when the polymerization or the copolymerization is carried out using a reaction solvent, its reaction system may be a homogeneous system or an heterogeneous system. Various solvents are operable, for example, there are included water, tetrahydrofuran, ether, 1,2-dimethoxyethane, acetonitrile, benzonitrile, acetone, methanol, ethanol, isopropanol, ethylene glycol, 2-methoxyethanol, 1-methyl-2-pyrrolidinone, dimethylformamide, dimethyl sulfoxide, acetic acid, hexane, pentane, benzene, toluene, xylene, dichloromethane, chloroform, carbon tetrachloride and the like. The solvents may be used alone or in combination of two or more kinds.

The polymerizable monomer in the copolymerization can use various compounds, for example, there are exemplified monomers having a carbon-carbon double bond or a carbon-carbon triple bond such as acetylene, ethylene, propylene, styrene, 1,3-butadiene, maleimide, N,N'-1,4-phenylenedimaleimide, (meth)arylic acid, sodium (meth)acrylate, (meth)acrylic ester, (meth)acrylonitrile, (meth)acrolein, (meth)acryloamide, vinylpyridine, vinylimidazole, vinylphosphonic acid, vinyltriethoxysilane, p-vinylbenzoic acid, sodium p-styrenesulfonate, p-styrenesulfonic acid, N-vinylpyrrolidone, vinylphenol and divinylbenzene; and ring-opening polymerizable monomers such as pyrrole, furan, thiophene, cyclopropane, cyclopropene, cyclopropanone, cyclopropenone, cyclobutene, ethylene oxide, oxetane, tetrahydrofuran and ethylenesulfide.

The copolymerization is carried out by polymerizing at least one kind of the multinuclear complex with one or more of other polymerizable monomers. The copolymerization can be carried out by combining various polymerizable monomers in various monomer ratios.

As manners of the polymerization treatment and the copolymerization treatment, regarding a polymerization initiation method, various techniques such as heat, light, electrolysis, radiation and oxidation can be used, and a radical generation catalyst and a radical initiator may be used. Ion polymerization may also be adopted using a cation generation catalyst or an anion generation catalyst. Among them, thermal polymerization and radical addition polymerization using a radical initiator are preferable.

Reactive conditions in the thermal polymerization of a preferable polymerization technique are explained.

The temperature range of the thermal polymerization is preferably 50°C or more and less than 350°C, more preferably 80°C or more and less than 250°C, and further preferably 80°C or more and less than 200°C. Regarding a gas atmosphere in conducting the thermal polymerization, the polymerization can be carried out under various gas atmospheres such as helium, argon, hydrogen, air, oxygen, carbon monoxide, water vapor and ammonia, and, nitrogen, helium and argon are preferable.

Reaction conditions in addition polymerization of another preferred embodiment using a radical initiator are explained.

As the radical initiator, there can be used an organic peroxide such as benzoyl peroxide, an inorganic peroxide such as potassium persulfate, or an azo based initiator such as 2,2'-azobisisobutyronitrile.

The polymerization temperature is determined by a radical generation temperature of a radical initiator used, preferably is 10°C or more, and 180°C or less. The polymerization time differs depending on the kind of polymerizable monomer and the polymerization temperature, but may be from about 0.5 hours to 24 hours.

The mode of reaction may be any of bulk polymerization, solution polymerization, suspension polymerization or emulsion polymerization. However, in cases of the suspension polymerization and the emulsion polymerization, if necessary, additives are sometimes used concomitantly, including water-soluble polymers such as polyvinyl alcohol, polyacrylic acid, polymethacrylic acid, gelatin, tragacanth, methyl cellulose and polymethacrylamide; talc, bentonite, sillic acid, diatom earth, clay, $BaSO_4$, $Al(OH)_3$, $CaSO_4$, $BaCO_4$, $MgCO_3$, $Ca(PO_4)_2$, $CaCO_3$, nonionic surfactants, anionic surfactants, cationic surfactants and ampholytic surfactants, these are used alone or in combination of two or more kinds.

As the additive, if necessary, a chain transfer agent can be also used concomitantly, including mercaptans such as t-dodecylmercaptan (TDM), n-dodecylmercaptan and n-octylmercaptan; α-methylstyrene dimer (αMSD) and terpinolenes.

The polymer obtained by polymerizing a multinuclear complex or the copolymer obtained by the multinuclear complex and other polymerizable monomer can be subjected to processing such as grinding, if necessary. As the grinding manner, grinding by a mortar, a agate mortar, a ball mill, a jet mill, a fine mill, a disc mill a hammer mill and the like can be included.

The multinuclear complex thus obtained, the polymer obtained by polymerizing the multinuclear complex, or the copolymer obtained by the multinuclear complex and other polymerizable monomer exhibits a unique catalyst activity of the multinuclear complex itself and an excellent stability, particularly heat stability, and it can be suitably used as a redox catalyst and the like.

Hereinafter, the present invention is specifically described based on Examples, but the present invention is not limited to the Examples.

Production example 1 [Synthesis of ligand]

A compound expressed by the following formula (6) (hereinafter called bbpr-allyl ligand) was synthesized in accordance with a synthesis of HL-Et ligand described in J. Am. Chem. Soc. 1984, 106, pp. 4765-4772. Namely, 2-Hydroxy-1,3-diaminopropane tetraacetic acid and o-diaminobenzene were reacted, then allylated using allyl chloride, thus to obtain bbpr-allyl ligand in a 71% yield. It was measured by $^1$H-NMR (0.05% (v/v) TMS $CDCL_3$ solution), as a result, introduction of an allyl group was confirmed by a peak of 4 to 6 ppm. Fig. 1 shows a $^1$H-NMR chart.

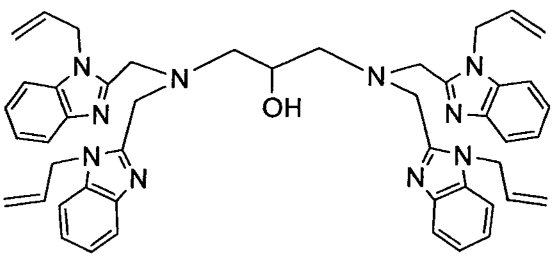

(6)

Production example 2 [Synthesis of ligand]

A ligand expressed by the following formula (7) can be produced in the same manner as Production example 1 in accordance with a synthesis of HL-Et ligand described in J. Am. Chem. Soc. 1984, 106, pp. 4765-4772, using epichlorohydrin in place of allyl chloride of Production example 1.

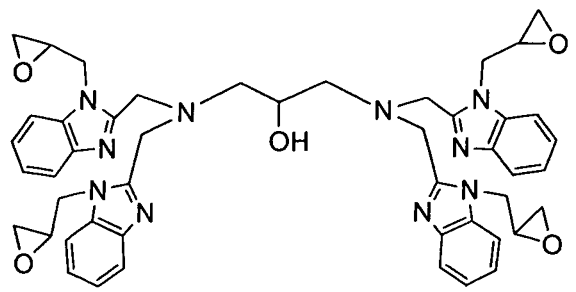

(7)

Example 1 [Production of multinuclear complex]

A multinuclear complex (hereinafter called Mn-(bbpr-allyl)-OTf) was synthesized in accordance with a method described in J. Am. Chem. Soc. 1994, 116, pp. 891-897. Namely, the bbpr-allyl ligand obtained in Production example 1 was mixed with manganese acetate tetrahydrate in an aqueous alcohol solution containing acetic acid and sodium acetate, further mixed with sodium triflate to obtain Mn-bbpr-allyl-OTf (yield 80%).
Element analysis, Calculated for $C_{51}H_{52}F_6Mn_2N_{10}O_9S_2$: C, 49.52; H, 4.24; N, 11.32; Found: C, 49.55; H, 4.37; N, 11.71.

Example 2 [Production of copolymer]

Mn-(bbpr-allyl)-OTf (30.1 mg, 0.024 mmol) and N,N'-1,4-phenylenedimaleimide (hereinafter abbreviated as PDM) (30.0 mg, 0.111 mmol) were mixed in an agate mortar, and this mixture was heat polymerized under the following conditions.

| | |
|---|---|
| Apparatus: | Rigaku TG8101D TAS200 |
| Gas atmosphere: | nitrogen |
| Temperature range: | 40°C to 300°C |
| Rising speed: | 10°C/min |
| Sample container: | open-type sample container made of aluminum ($\phi$ 5.2 mm, H 5.0 mm, 100 $\mu$l) |
| Amount of sample: | the mixture 16$\pm$2 mg/the sample container. |

According to the above, (Mn-(bbpr-allyl)-OTf/PDM) copolymer was obtained. The manganese element analysis of the copolymer was conducted (sulfuric acid/nitric acid decomposition -hydrochloric acid dissolution- ICP optical emission spectrometry) to find the content of manganese of 4.21 wt%.

Example 3 [Hydrogen peroxide decomposition test of copolymer]

The (Mn-(bbpr-allyl)-OTf)/PDM) copolymer obtained in Example 2 (10.98 mg, 8.41 $\mu$mol (per one metal atom)) was weighed in a two neck flask of 25 ml. Herein, as a solvent, a solution (1.00 ml) of poly(sodium 4-styrenesulfonate) (commercial product of Aldrich, weight-average molecular weight: about 70, 000) dissolved in tartaric acid/sodium tartarate buffer solution (prepared with 0.20 mol/l aqueous tartaric acid solution and 0.10 mol/l aqueous sodium tartarate solution, pH 4.0) to a polymer concentration of 21.1 mg/ml was added to the flask, subsequently ethylene glycol (1.00 ml) was added and stirred. This was used as a catalyst mixed solution.

A septum was equipped to one neck of the two-neck flask containing this catalyst mixed solution, and the other neck was connected to a gas bullet. After this flask was stirred at 80°C for 5 minutes as heat treatment before reaction, aqueous hydrogen peroxide solution (11.4 mol/l, 0.20 ml (2.28 mmol)) was added with a syringe, and hydrogen peroxide decomposition reaction was conducted at 80°C for 20 minutes. Oxygen being generated was measured with the gas bullet, and the quantity of decomposed hydrogen peroxide was measured. The measured volume (v) that the generated oxygen was measured with the gas bullet was converted to the conditions of 0°C, 101325 Pa (760 mmHg) by Numerical expression 1 taking atmospheric pressure and water vapor pressure into consideration, thereby obtaining a gas yield (V).

$$V = [273v(P-p)]/[760(273+t)] \quad \text{(Numerical expression 1)}$$

in the Numerical expression 1, represented are P: atmospheric pressure (mmHg), p: vapor pressure of water (mmHg),

t: temperature (°C), v: measured volume (ml), V: volume (ml) at 0°C, 101325 Pa (760 mmHg).

Fig. 2 shows variation with time of the amount of oxygen generated (elapsed time is t).

Thereafter, the reaction solution was diluted with a water/acetonitrile mixed solution (water: acetonitrile = 7:3, (v/v)) for the solution volume to be 10.0 ml, and this solution was filtered through a syringe filter. The filtrate was subjected to GPC measurement (GPC analysis conditions are as follows), and the weight-average molecular weight of poly(sodium 4-styrenesulfonate) after the test was obtained. By comparing this weight-average molecular weight after the test with the weight-average molecular weight of poly(sodium 4-styrenesulfonate) before the test, the degree of lowering molecular weight of the polymer due to free radicals derived from hydrogen peroxide was examined, thereby estimating the amount of free radicals generated.

Table 1 shows the result of weight-average molecular weights.

GPC (gel permeation chromatography) analysis conditions

| | |
|---|---|
| Column: | TSK gel α-M manufactured by Tosoh Corporation (13 μm, 7.8 mmϕ × 30 cm) |
| Column temperature: | 40°C |
| Mobile phase | 50 mmol/l aqueous ammonium acetate solution: $CH_3CN$, =7:3 (v/v) |
| Flow rate: | 0.6 ml/min |
| Detector: | RI |
| Injection amount: | 50 μl |
| Molecular weight determination: | a weight-average molecular weight was obtained in terms of polyethylene oxide conversion value. |

Measurement of weight-average molecular weight of poly(sodium 4-styrenesulfonate) before test

The weight-average molecular weight of poly(sodium 4-styrenesulfonate) (21.1 mg, commercial product of Aldrich) was measured in the same manner as the GPC analysis conditions.

From Table 1, the weight-average molecular weight of poly(sodium 4-styrenesulfonate) coexisted in Example 3 was almost the same as that in the product before the test. From this fact, it was made clear that the catalyst of Example 3 suppressed the generation of free radicals and decomposed hydrogen peroxide.

Example 4 [Hydrogen peroxide decomposition test of copolymer]

The same test as Example 3 was conducted in the same manner except that the heat treatment before reaction was set to a stirring condition at 80°C for 60 minutes. Fig. 2 shows variation with time of the converted amount of oxygen generated, and Table 1 shows the weight-average molecular weight after the test.

The hydrogen peroxide decomposition test was conducted using the copolymer obtained in Example 2 as a catalyst, as a result, it was made clear that the catalyst has high heat-stability since the catalyst activity is not deteriorated at all even being subjected to hot water pretreatment for 1 hour.

From Table 1, the weight-average molecular weight of poly(sodium 4-styrenesulfonate) coexisted in Example 4 was almost the same as that in the product before the test. From this fact, it was made clear that the catalyst of Example 4 which was subjected to hot water pretreatment for 1 hour suppressed the generation of free radicals and decomposed hydrogen peroxide.

Table 1

| Sample | Weight-average molecular weight |
|---|---|
| Example 3 (Heat treatment before reaction: 5 min) | $1.0\times10^5$ |
| Example 4 (Heat treatment before reaction: 60 min) | $9.6\times10^4$ |
| Product before test | $1.1\times10^5$ |

Example 5 [Production of multinuclear complex]

Using the ligand expressed by the formula (7) shown in Production example 2, binuclear manganese complex of the ligand L having an epoxy ring can be obtained in the same manner as in Example 1.

Production example 3 [Synthesis of ligand]

A bbpr-$CH_2$St ligand expressed by the following formula (8) was obtained in a 85% yield in accordance with a synthesis of HL-Et ligand described in J. Am. Chem. Soc. 1984, 106, pp. 4765-4772, in the same manner as Production example 1 using 4-chloromethylstyrene in place of allyl chloride. It was measured by $^1$H-NMR (0.05% (v/v) TMS $CDCL_3$ solution), as a result, introduction of a -$CH_2$St group was confirmed by a peak of 5 to 8 ppm. Fig. 3 shows a $^1$H-NMR chart.

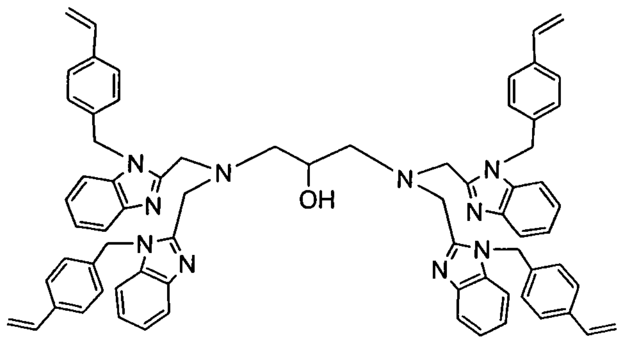

(8)

Production example 4 [Synthesis of multinuclear complex precursor]

In a flask, p-vinylbenzoic acid (10.1 g, 67.5 mmol) and aqueous sodium hydroxide solution (10.2 g, 64.1 mmol) were weighed, 140 ml of water was added thereto, stirred and dissolved, the undissolved component was filtered off, thereby preparing an aqueous sodium p-vinylbenzoate solution. Separately, manganese (II) sulfate pentahydrate (7.74 g, 32.1 mmol) and 50 ml of water were weighed in a flask, stirred and dissolved. The aqueous sodium p-vinylbenzoate solution was added thereto, and stirred at room temperature for 2 hours. The precipitation produced was collected by filtration, washed with water, washed with ether, and then dried under reduced pressure to obtain a white powder of manganese p-vinylbenzoate tetrahydrate. Yield 5.87 g (13.9 mmol) 43%, element analysis, Calculated for $C_{18}H_{22}MnO_8$: C, 51.32; H, 5.26, Found: C, 51.63, H, 5.16.

Example 6 [Production of multinuclear complex]

In a flask, bbpr-$CH_2$St (1.12 g, 1.04 mmol), sodium acetate (324 mg, 11.8 mmol) and acetic acid (120 mg, 3.95 mmol) were weighed, 120 ml of dimethyl sulfoxide was added thereto, stirred and dissolved. Manganese acetate tetrahydrate (637 mg, 2.60 mmol) was added thereto, and stirred at room temperature for 1.5 hours. Thereafter, sodium triflate (447 mg, 2.60 mmol) was added, and further stirred for 1 hour. This reaction mixture was poured into a beaker having 700 ml of distilled water, the precipitation produced was collected by filtration. The precipitation was washed with water, and then dried under reduced pressure, thereby obtaining a white powder of Mn-OAc- (bbpr-$CH_2$St) -OTf expressed by the following formula (9). Yield 1.22 g (0.792 mmol, 76%). ESI MS [M-$CF_3SO_3$] + = 1391.2.

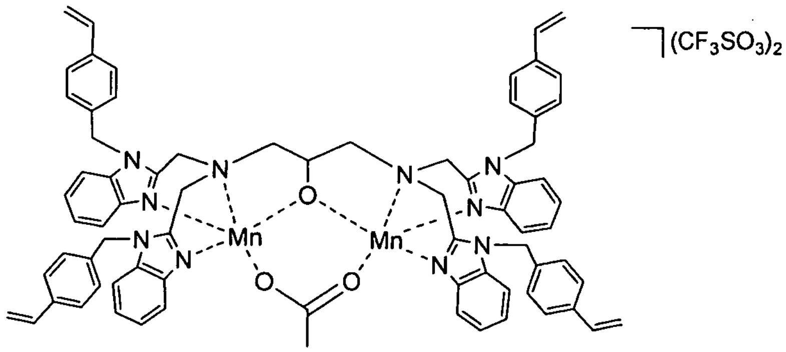

(9)

Example 7 [Production of multinuclear complex]

In a flask, bbpr-$CH_2$St (400 mg, 0.372 mmol) and diisopropylethylamine (43.2 mg, 0.335 mmol) were weighed, 54 ml of tetrahydrofuran was added thereto, stirred and dissolved. Manganese p-vinylbenzoate tetrahydrate (313 mg, 0.744 mmol) was added thereto, and stirred at room temperature for 2 hours. This reaction mixture was concentrated under reduced pressure, methanol was added, and the precipitation produced was collected by filtration, washed with water, washed with ether, and then dried under reduced pressure, thereby obtaining a beige powder of Mn-vb-(bbpr-$CH_2$St)-vb expressed by the following formula (10). Yield 122 mg. ESI MS [M-(p-vinylbenzoate )]$^+$ = 1477.4.

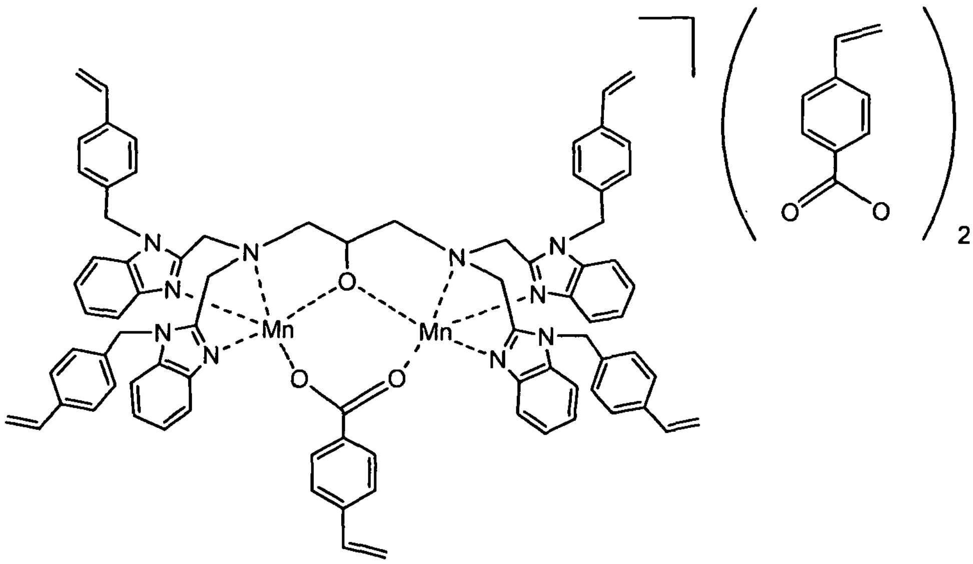

(10)

Example 8 [Production of copolymer]

In a 10 ml sample tube made of glass, Mn-OAc-(bbpr-$CH_2$St)-OTf (770 mg, 0.500 mmol), sodium p-styrenesulfonate hydrate (35.1 mg), N-vinylpyrrolidone (299 mg, 2.69 mmol), dimethylformamide (391 mg) and 1,1'-azobis(cyclohexane-1-carbonitrile) (39.7 mg, 0.162 mmol) were mixed and dissolved. After argon gas was flowed in this sample tube, which was sealed with a rubber septum, and the mixture was heated and polymerized at 80°C in an oil bath for 24 hours. A gel-like copolymer produced was taken out by crashing the sample tube. The gel-like copolymer taken out was ground with a hammer and an agate mortar to obtain a light yellow powder (1.29 g, yield: the content of manganese is 0.775 μmol/mg provided that manganese in a starting material is contained by 100%).

Example 9 [Hydrogen peroxide decomposition test of copolymer]

The copolymer catalyst obtained in Example 8 (21.6 mg, 20.0 μmol (per one manganese atom, calculated by the content of manganese)) as a hydrogen peroxide decomposition catalyst, and tartaric acid/sodium tartarate buffer solution (2.00 ml, prepared by 0.20 mol/l aqueous tartaric acid solution and 0.10 mol/l aqueous sodium tartarate solution;

pH 4.0) were weighed in a two neck flask. This was used as a catalyst mixed solution.

Using this catalyst mixed solution, the hydrogen peroxide decomposition reaction was conducted for 20 minutes in the same manner as Example 3, oxygen generated was measured with a gas bullet to determine the quantity of decomposed hydrogen peroxide. Fig. 4 shows variation with time of the amount of oxygen generated (elapsed time is t).

Example 10 [Hydrogen peroxide decomposition test of copolymer]

The same test as Example 9 was conducted in the same manner except that the heat treatment before reaction was set to the stirring condition at 80°C for 6 hours. Fig. 4 shows variation with time of the converted amount of oxygen generated.

The hydrogen peroxide decomposition test was conducted using the copolymer obtained in Example 8 as a catalyst, as a result, it was made clear that deterioration of catalyst activity was small even being subjected to hot water pretreatment for 6 hours and the catalyst had high heat-stability.

Example 11 [Production of copolymer]

In a 10 ml sample tube made of glass, Mn-vb- (bbpr-$CH_2$St) -vb (100 mg, 0. 0615 mmol), N-vinylimidazole (68.4 mg, 0.723 mmol), acrylic acid (51.6 mg, 0.716 mmol), tetrahydrofuran (330 mg) and 2,2'-azobis(2,4-dimethylvaleronitrile) (9.20 mg, 0.0370 mmol) were mixed and dissolved. After argon gas was flowed in this sample tube, which was sealed with a rubber septum, and the mixture was heated and polymerized at 50°C in an oil bath for 16 hours. A gel-like copolymer produced was taken out by crashing the sample tube. The gel-like copolymer taken out was ground with a hammer and an agate mortar to obtain a white powder (187 mg).

Example 12 [Production of copolymer]

In a 10 ml sample tube made of glass, Mn-vb- (bbpr-$CH_2$St) -vb (100 mg, 0. 0615 mmol), methacrylonitrile (62.0 mg, 0.924 mmol), acrylic acid (11.5 mg, 0.160 mmol) and 2,2'-azobis(2,4-dimethylvaleronitrile) (9.20 mg, 0.0370 mmol) were mixed and dissolved. This was subjected to polymerization and grinding treatment in the same manner as in Example 11 to obtain a white powder of copolymer (99.0 mg).

Example 13 [Production of copolymer]

In a 10 ml sample tube made of glass, Mn-vb-(bbpr-$CH_2$St)-vb (100 mg, 0.0615 mmol), methacrylonitrile (82.0 mg, 1.22 mmol), acrylic acid (23.1 mg, 0.321 mmol), divinylbenzene (41.0 mg, 0.315 mmol) and 2,2'-azobis(2,4-dimethylvaleronitrile) (9.20 mg, 0.0370 mmol) were mixed and dissolved. This was subjected to polymerization and grinding treatment in the same manner as Example 11 to obtain a white powder of copolymer (143 mg).

Example 14 [Production of copolymer]

In a 2 ml sample tube made of glass, Mn-vb- (bbpr-$CH_2$St) -vb (100 mg, 0.0615 mmol), acryloamide (20.4 mg, 0.287 mmol), methacrylic acid (73.5 mg, 0.854 mmol) and 2,2'-azobis(2,4-dimethylvaleronitrile) (9.20 mg, 0.0370 mmol) were mixed and dissolved. This was subjected to polymerization and grinding treatment in the same manner as Example 11 to obtain a white powder of copolymer (143 mg).

Example 15 [Production of copolymer]

In a 2 ml sample tube made of glass, Mn-vb- (bbpr-$CH_2$St) -vb (100 mg, 0.0615 mmol), methacryloamide (21.0 mg, 0.247 mmol), methacrylic acid (73.5 mg, 0.854 mmol) and 2,2'-azobis(2,4-dimethylvaleronitrile) (9.20 mg, 0.0370 mmol) were mixed and dissolved. This was subjected to polymerization and grinding treatment in the same manner as Example 11 to obtain a white powder of copolymer (130 mg).

Example 16 [Production of copolymer]

In a 2 ml sample tube made of glass, Mn-vb- (bbpr-$CH_2$St) -vb (200 mg, 0.123 mmol), methacryloamide (60.0 mg, 0.705 mmol), acrylic acid (49.3 mg, 0.684 mmol), methacrolein (135 mg, 1.93 mmol) and 2,2'-azobis(2,4-dimethylvaleronitrile) (18.4 mg, 0.0740 mmol) were mixed and dissolved. This was subjected to polymerization and grinding treatment in the same manner as Example 11 to obtain a white powder of copolymer (130 mg).

Example 17 [Production of multinuclear complex]

In a flask, Mn-vb-(bbpr-$CH_2$St)-vb (500 mg, 0.308 mmol) and sodium n-dodecylbenzenesulfonate (215 mg, 0.616 mmol) were each weighed, and dissolved in tetrahydrofuran (30 ml). This was stirred for 2 hours, and then the solvent was removed under reduced pressure. The resulting residue was washed with hexane and water, and then dried in vacuum, thereby obtaining Mn-vb-(bbpr-$CH_2$St) -DBS expressed by the following formula (11) having two n-dodecylbenzenesulfonates as counter anions. Yield 481 mg (0.242 mmol, 62%).

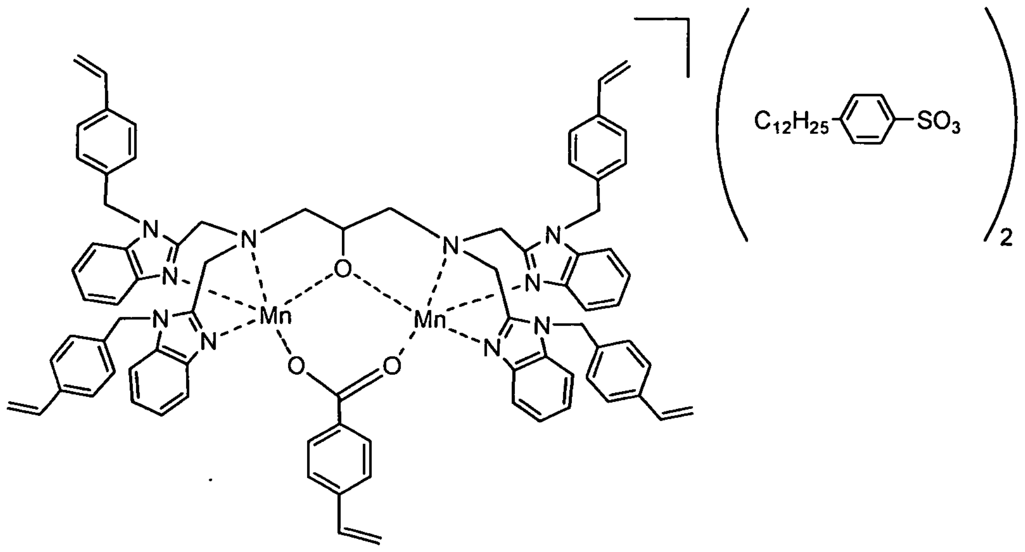

(11)

Example 18 [Production of copolymer]

Mn-vb-(bbpr-$CH_2$St)-DBS (100 mg, 0.0509 mmol), methacrylonitrile (166 mg, 2.47 mmol), methacrolein (95.1 mg, 0.730 mmol), toluene (150 mg), Aquaron HS-10 (169 mg, manufactured by Daiichi Kogyo Seiyaku Co., Ltd), 2, 2' -azobis (2, 4-dimethylvaleronitrile) (18. 0 mg, 0.0720 mmol) and distilled water (5 ml, bubbled with nitrogen gas for 30 minutes) were weighed in a 25 ml flask equipped with a rotor of 1.2 mm after being replaced with nitrogen, the mixture was stirred at 450 rpm, and polymerized at 50°C for 3 hours.

Methanol was added to the reaction mixture obtained to precipitate, and the precipitation was collected by filtration and washed with methanol, and then dried in vacuum. (Yield 148 mg).

Example 19 [Production of multinuclear complex]

In a flask, bbpr-$CH_2$St (1.46 g, 1.36 mmol), diisopropylethylamine (0.160 g, 1.24 mmol) and cobalt acetate tetrahydrate (0.686 mg, 2.75 mmol) were each weighed, and dissolved in dimethyl sulfoxide (50 ml). This was stirred for 1 hour, and then sodium tetraphenylborate (0.941 mg, 5.50 mmol) was added thereto, and stirred for 30 minutes. Water was added to this reaction mixture, the precipitation produced was collected by filtration, washed with water, washed with ether, and then dried in vacuum to obtain Co-(bbpr-$CH_2$St)-$BPh_4$ expressed by the following formula (12). Yield 2.48 g (62%). ESI MS $[M-(BPh_4)]^+ = 1570.6$.

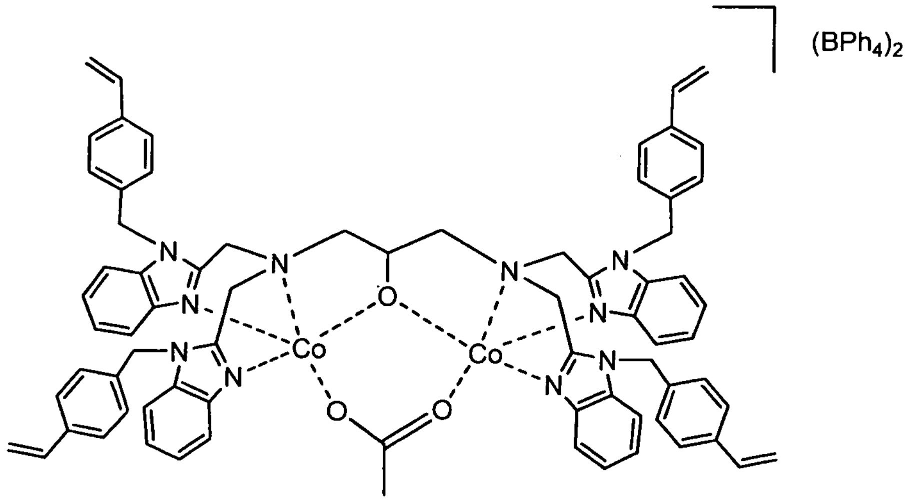

(12)

Example 20 [Production of multinuclear complex]

By conducting complex formation reaction in the same manner as Example 19 except use of nickel acetate tetrahydrate (0.687 mg, 2.75 mmol) in place of cobalt acetate tetrahydrate (0.686 mg, 2.75 mmol), Ni-(bbpr-$CH_2$St)-$BPh_4$ expressed by the following formula (13) was obtained. Yield 2.55 g (62%). ESI Ms $[M-(BPh_4)]^+$ = 1568.5.

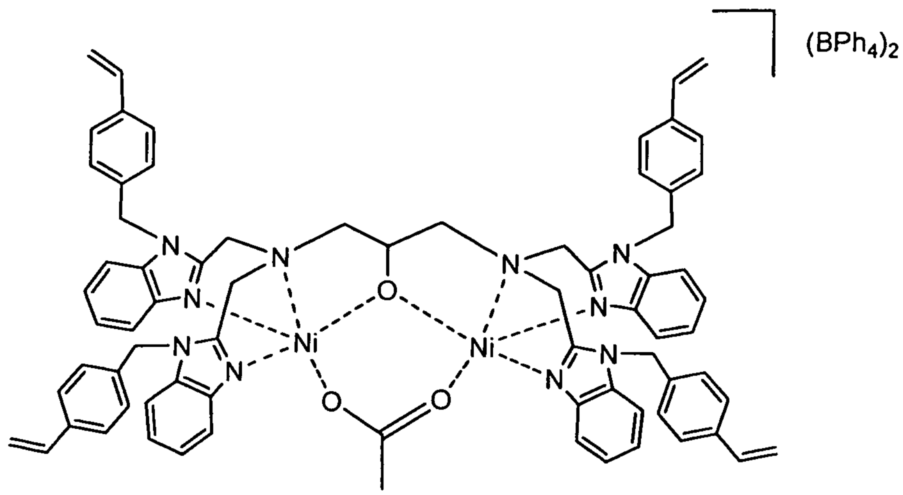

(13)

Example 21 [Production of multinuclear complex]

By conducting complex formation reaction in the same manner as Example 19 except use of copper (II) acetate monohydrate (0.549 mg, 2.75 mmol) in place of cobalt acetate tetrahydrate (0.686 mg, 2.75 mmol), Cu-(bbpr-$CH_2$St)-$BPh_4$ expressed by the following formula (14) was obtained. Yield 2.39 g (78%).

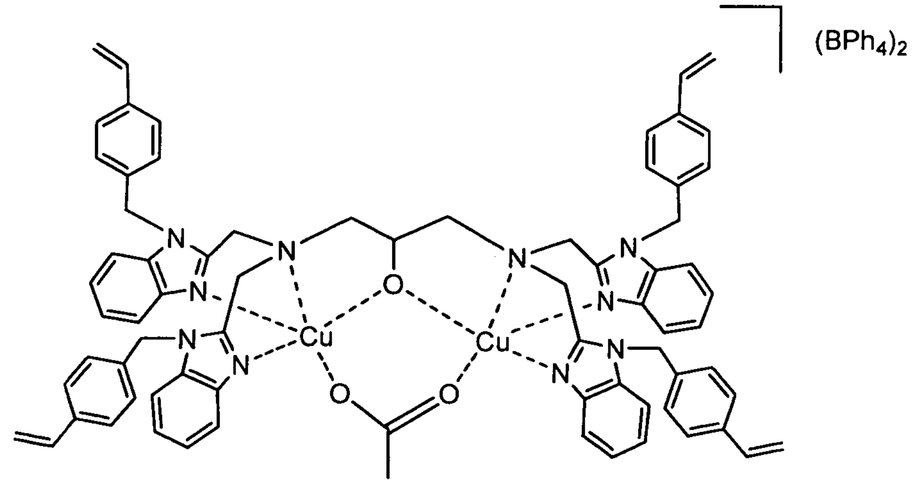

(14)

Example 22 [Production of multinuclear complex]

By conducting complex formation reaction in the same manner as Example 19 except use of iron (II) chloride tetrahydrate (0.545 mg, 2.77 mmol) in place of cobalt acetate tetrahydrate (0.686 mg, 2.75 mmol), Fe-(bbpr-$CH_2$St)-$BPh_4$ expressed by the following formula (15) was obtained. Yield 2.77 g (62%).

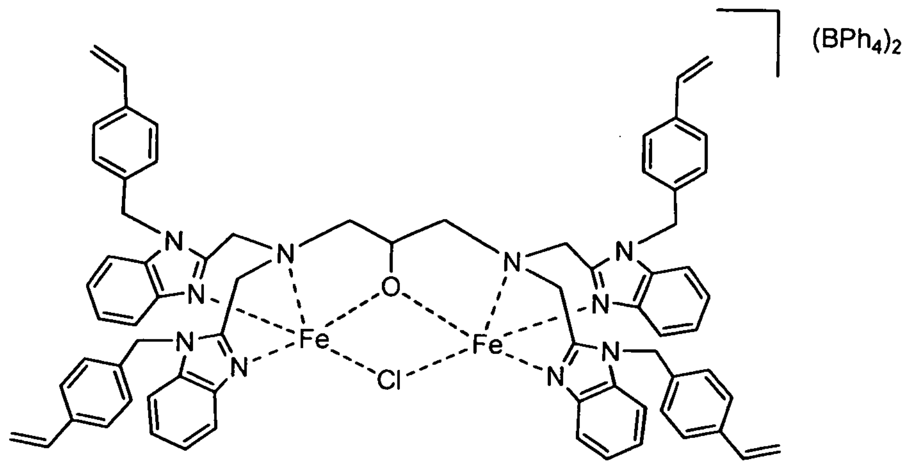

(15)

The multinuclear complex of the present invention and the polymer obtained by polymerizing the multinuclear complex are useful as a redox catalyst, in particular, when the polymer and the copolymer are used as a hydrogen peroxide decomposition catalyst, it is possible to decompose hydrogen peroxide into water and oxygen while suppressing the generation of free radicals, in comparison with the multinuclear complex catalyst disclosed so far, showing significantly high stability. Such a catalyst can be used in applications such as an antidegradant for polyelectrolyte type fuel cells and water electrolysis equipment, an antioxidant for medicines, agricultural chemicals and foods.

## Claims

1. A multinuclear complex comprising at least one ligand L satisfying the following requirements (i), (ii) and (iii), and a plurality of metal atoms in a molecule:

    (i) having a group having polymerizable reactive multiple bonds, and/or a ring-opening polymerizable ring,
    (ii) having five or more coordination atoms bonding to the metal atoms, and
    (iii) being soluble in a solvent.

**2.** The multinuclear complex according to claim 1, wherein a coordination atom of the ligand L is a nitrogen atom, an oxygen atom, a phosphorous atom or a sulfur atom.

**3.** The multinuclear complex according to claim 1, wherein at least one of coordination atoms of the ligand L is a nitrogen atom having a double bond with carbon.

**4.** The multinuclear complex according to claim 1, wherein the total of metal atoms contained in a molecule is 8 or less.

**5.** The multinuclear complex according to claim 1, wherein a metal atom contained in a molecule is a transition metal atom of the first transition element series.

**6.** The multinuclear complex according to claim 1, wherein two metal atoms selected from said plurality of metal atoms are coordinately bonded with the same coordination atom, or have a combination of $AM^1$ and $AM^2$ such that the minimum of a covalent bond linking $AM^1$ and $AM^2$ is 1 or more, and 4 or less, when two metal atoms selected from a plurality of metal atoms are denoted as $M^1$, $M^2$, and coordination atoms bonding to $M^1$ and $M^2$ are denoted as $AM^1$ and $AM^2$, respectively.

**7.** The multinuclear complex according to claim 1, wherein the ligand L is one, and the metal atoms are two.

**8.** The multinuclear complex according to claim 1, wherein the molecular weight is 6000 or less.

**9.** A compound expressed by the following formula (1):

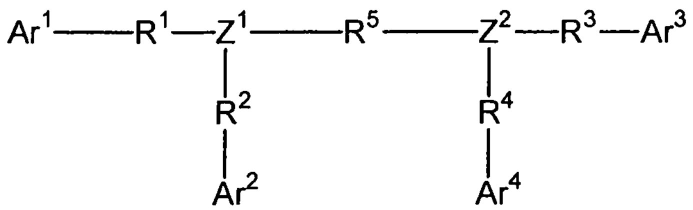

(1)

wherein $Ar^1$, $Ar^2$, $Ar^3$ and $Ar^4$ (hereinafter sometimes denoted as $Ar^1$ to $Ar^4$) each independently represents an aromatic heterocyclic group, $R^1$, $R^2$, $R^3$, $R^4$ and $R^5$ (hereinafter sometimes denoted as $R^1$ to $R^5$) represent a divalent group, $Z^1$ and $Z^2$ each independently represents a nitrogen atom or a trivalent group; at least one of $Ar^1$ to $Ar^4$ and $R^1$ to $R^5$ has a group having polymerizable reactive multiple bonds, and/or a ring-opening polymerizable ring.

**10.** The compound according to claim 9, expressed by the following formula (2):

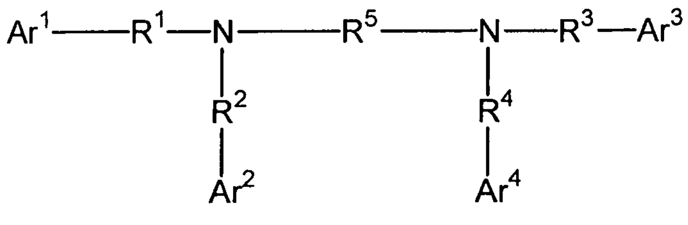

(2)

wherein $Ar^1$ to $Ar^4$, and $R^1$ to $R^5$ are the same meanings as in said formula (1), and at least one of them has a group having polymerizable reactive multiple bonds, and/or a ring-opening polymerizable ring.

**11.** The compound according to claim 10, expressed by the following formula (3a) or (4a):

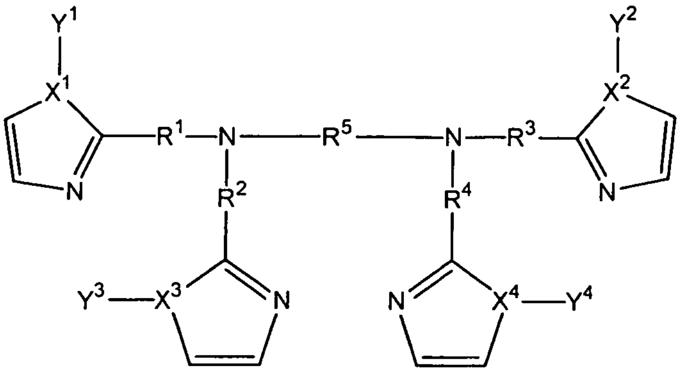

(3a)

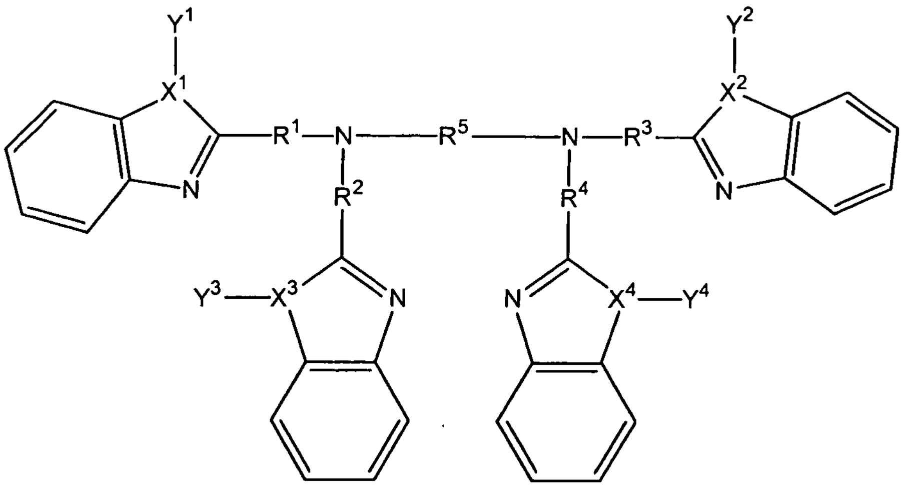

(4a)

In the formulas (3a) and (4a), $R^1$ to $R^5$ are the same meanings as in the formula (1). $X^1$, $X^2$, $X^3$ and $X^4$ (hereinafter sometimes denoted as $X^1$ to $X^4$) are selected from a nitrogen atom or CH. $Y^1$, $Y^2$, $Y^3$ and $Y^4$ (hereinafter sometimes denoted as $Y^1$ to $Y^4$) represent a hydrogen atom, an alkyl group having 1 to 50 carbon atoms, an aromatic group having 2 to 60 carbon atoms, a group having a polymerizable reactive carbon-carbon double bond or a group having a ring-opening polymerizable ring, and at least one of $Y^1$ to $Y^4$ is a group having a polymerizable reactive carbon-carbon double bond or a group having a ring-opening polymerizable ring.

**12.** The compound according to claim 11, expressed by the following formula (3b) or (4b):

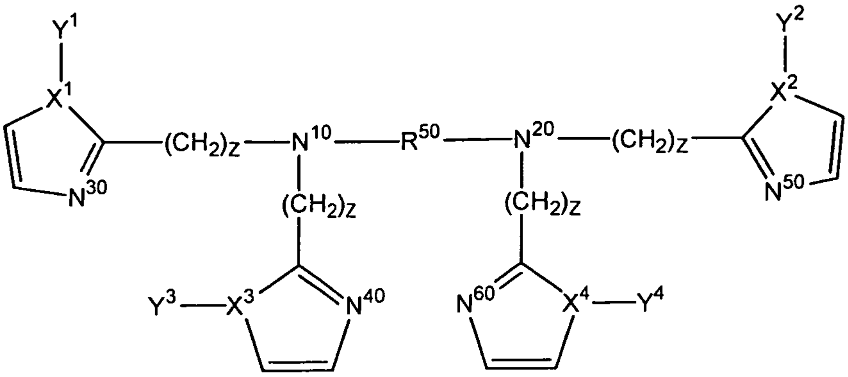

(3b)

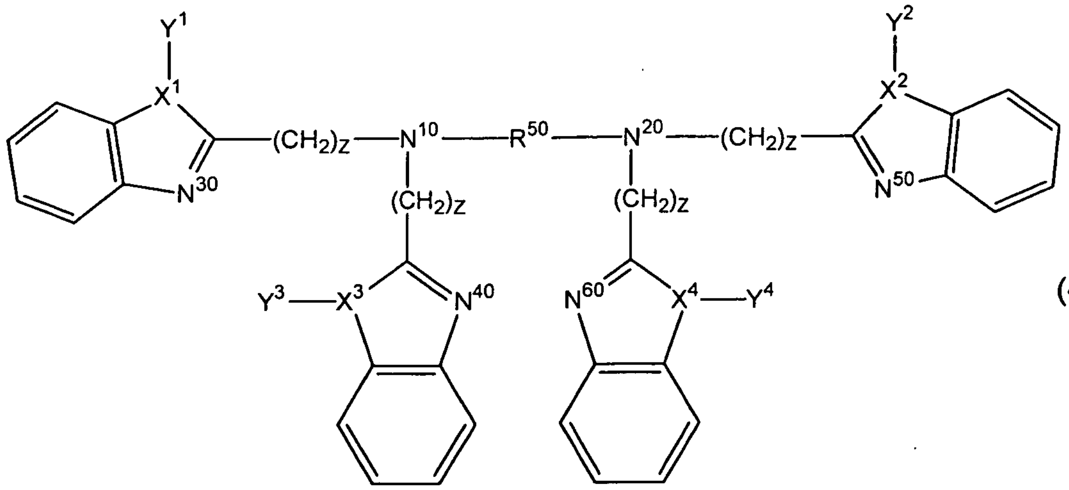

(4b)

In the formulas (3b) and (4b), $X^1$, $X^2$, $X^3$ and $X^4$ (hereinafter sometimes denoted as $X^1$ to $X^4$), and $Y^1$, $Y^2$, $Y^3$ and $Y^4$ (hereinafter sometimes denoted as $Y^1$ to $Y^4$) are the same meanings as in the formula (3a) or (4a). At least one of $Y^1$ to $Y^4$ is a group having a polymerizable reactive carbon-carbon double bond or a group having a ring-opening polymerizable ring. Z represents an integer of 1 or 2. $N^{10}$ and $N^{20}$ represent a nitrogen atom bonding with $R^{50}$, and $N^{30}$, $N^{40}$, $N^{50}$ and $N^{60}$ (hereinafter sometimes denoted as $N^{30}$ to $N^{60}$) represent a nitrogen atom in an aromatic heterocyclic group. $R^{50}$ represents a divalent group having the minimum of a covalent bond linking $N^{10}$ and $N^{20}$ of 2 or more, and 14 or less.

**13.** The compound according to claim 12, expressed by the following formula (3c) or (4c):

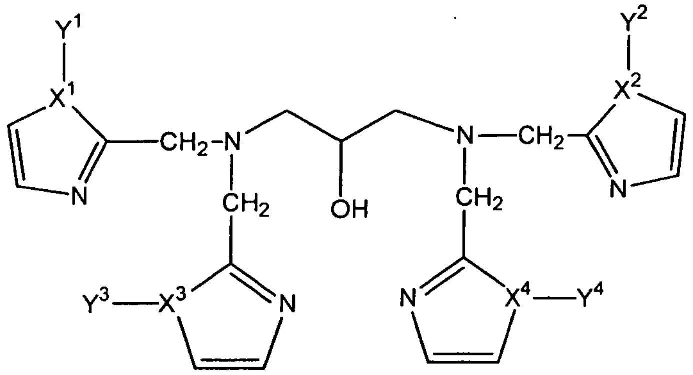

(3c)

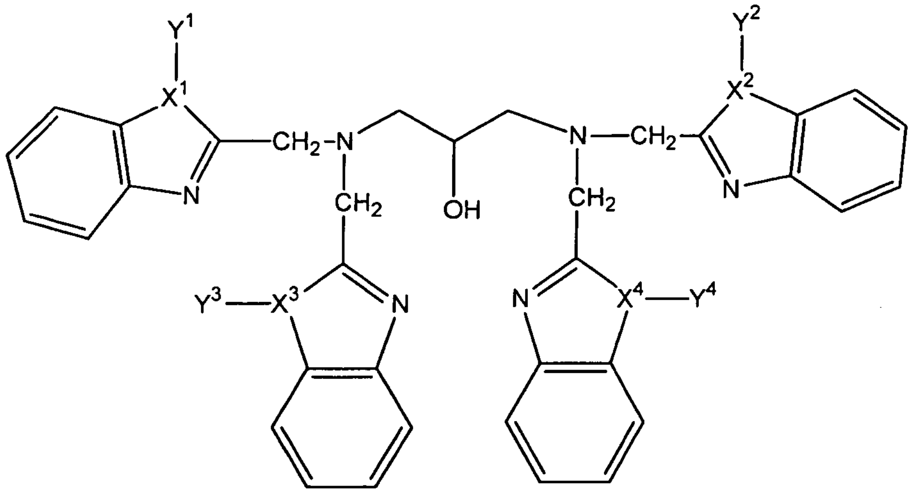

(4c)

In the formulas (3c) and (4c), $X^1$ to $X^4$, and $Y^1$ to $Y^4$ are the same meanings as in the formula (3a) or (4a), and at least one of $Y^1$ to $Y^4$ is a group having a polymerizable reactive carbon-carbon double bond or a group having a ring-opening polymerizable ring.

**14.** The multinuclear complex according to any one of claims 1 to 8, which the compound of any one of claims 9 to 13 as the ligand L.

**15.** A polymer obtained by polymerizing the multinuclear complex according to any one of claims 1 to 8 and 14.

**16.** A copolymer obtained by copolymerizing at least one kind of the multinuclear complex according to any one of claims 1 to 8 and 14 with a polymerizable monomer capable of copolymerizing with said multinuclear complex.

**17.** A redox catalyst using the multinuclear complex according to any one of claims 1 to 8 and 14, the polymer of claim 15, or the copolymer of claim 16.

Fig. 1

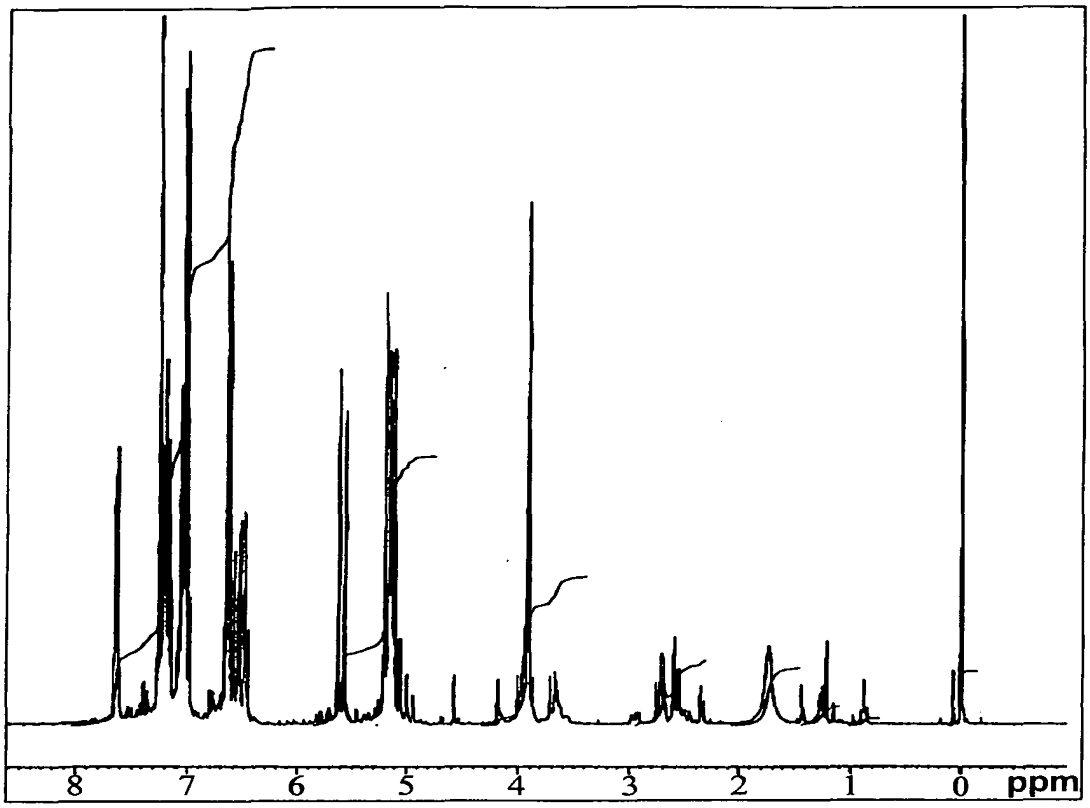


Fig.2

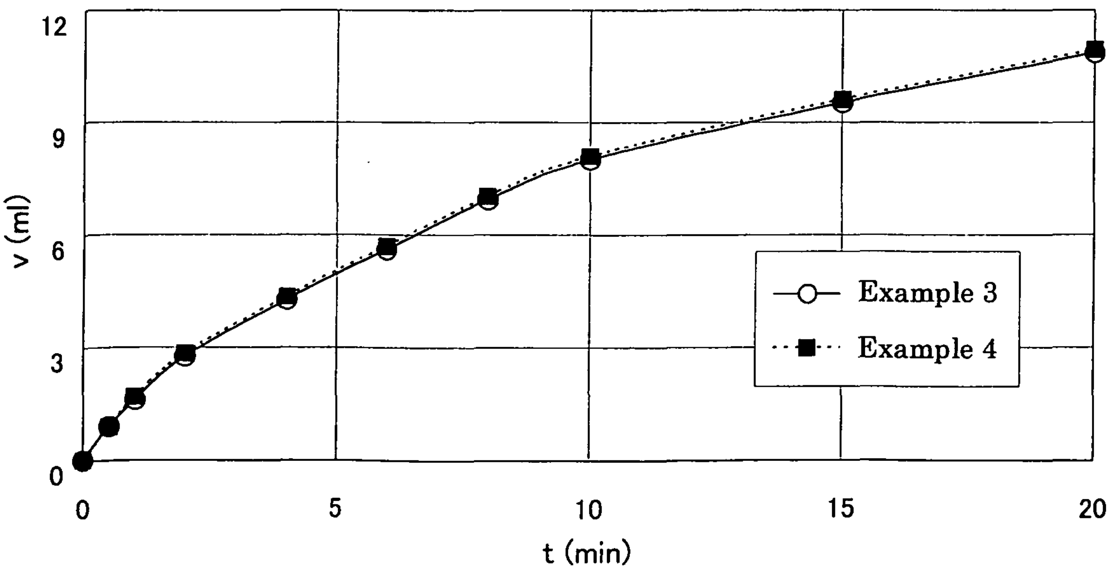

Fig.3

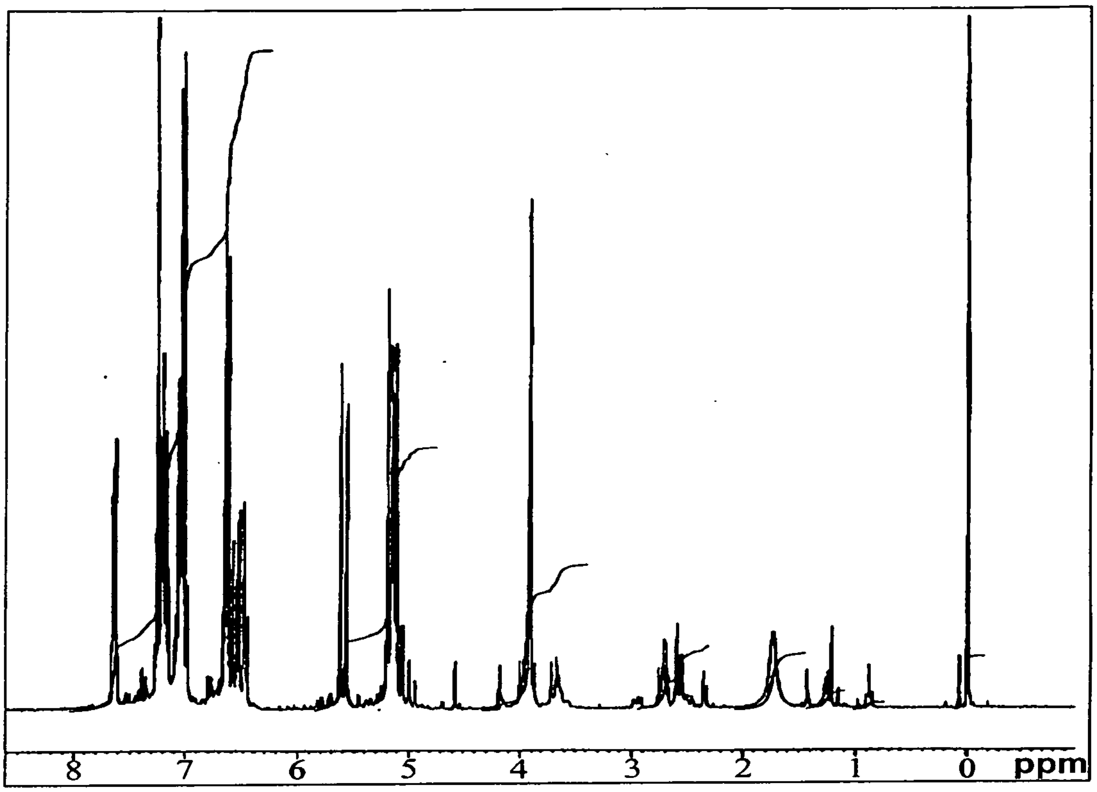

Fig.4

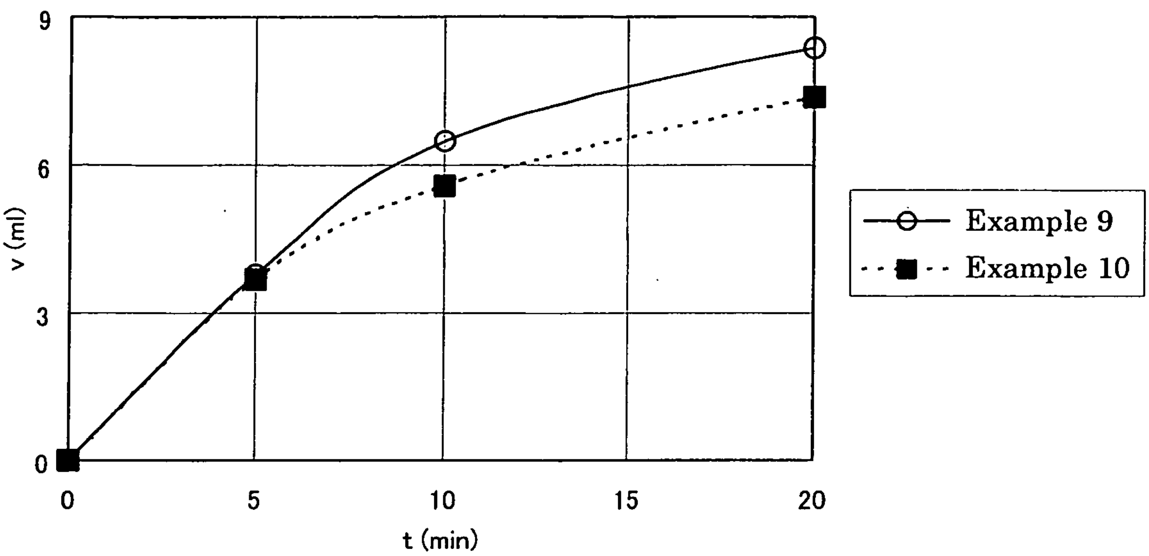

## INTERNATIONAL SEARCH REPORT

International application No.
PCT/JP2007/052540

A. CLASSIFICATION OF SUBJECT MATTER
*C07D235/14*(2006.01)i, *B01J31/22*(2006.01)i, *C07D405/06*(2006.01)i, *C08F4/40*(2006.01)i, *C08F12/26*(2006.01)i, *C08F26/02*(2006.01)i, *C07F1/08*(2006.01)n, *C07F13/00*(2006.01)n, *C07F15/02*(2006.01)n,
According to International Patent Classification (IPC) or to both national classification and IPC

B. FIELDS SEARCHED
Minimum documentation searched (classification system followed by classification symbols)
C07D235/14, B01J31/22, C07D405/06, C08F4/40, C08F12/26, C08F26/02, C07F1/08, C07F13/00, C07F15/02, C07F15/04, C07F15/06

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | Boelrijk A.E.M. et al., Mechanism of hydrogen peroxide dismutation by a dimanganese catalase mimic: Dominant role of an intramolecular base on substrate binding affinity and rate acceleration, Inorganic Chemistry, 2000, Vol.39, No.14, pp.3020-3028, particularly, abstract, Fig. 2 | 1-17 |
| A | EP 1531193 A2 (Henkel KGaA), 18 May, 2005 (18.05.05), Particularly, Claim 8 & DE 10345820 A1 | 1-17 |
| A | JP 10-156188 A (Lion Corp.), 16 June, 1998 (16.06.98), Particularly, Par. Nos. [0006], [0007] (Family: none) | 1-17 |

☒ Further documents are listed in the continuation of Box C.   ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 08 March, 2007 (08.03.07) | 20 March, 2007 (20.03.07) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

International application No.
PCT/JP2007/052540

C (Continuation). DOCUMENTS CONSIDERED TO BE RELEVANT

| Category* | Citation of document, with indication, where appropriate, of the relevant passages | Relevant to claim No. |
|---|---|---|
| A | Gavrilova A.L. et al., Principles of mononucleating and binucleating ligand design, Chemical Reviews, 2004, Vol.104, No.2, pp.349-383, particularly, Table 10 | 1-17 |
| A | WO 2006/008438 A1 (Innovene Europe Ltd.), 26 January, 2006 (26.01.06), Particularly, Claim 24 (Family: none) | 1-17 |
| P,A | WO 2007/000956 A1 (Sumitomo Chemical Co.), 04 January, 2007 (04.01.07), Particularly, pages 20 to 21 (Family: none) | 1-17 |

Form PCT/ISA/210 (continuation of second sheet) (April 2005)

**INTERNATIONAL SEARCH REPORT**

International application No.
PCT/JP2007/052540

Continuation of A. CLASSIFICATION OF SUBJECT MATTER
(International Patent Classification (IPC))

*C07F15/04*(2006.01)n, *C07F15/06*(2006.01)n

(According to International Patent Classification (IPC) or to both national classification and IPC)

Form PCT/ISA/210 (extra sheet) (April 2005)

INTERNATIONAL SEARCH REPORT

International application No.
PCT/JP2007/052540

<Regarding Coverage of Search>

Although claims 1-10 relate to an invention of a chemical substance, they fail to sufficiently specify the chemical structure of the substance. Consequently, claims 1-10 fail to satisfy the requirement of clearness prescribed under PCT Article 6.

In addition, among the chemical substances of claims 1-10, only the compounds represented by the formulae (6), (7), (8) and their complexes are specifically disclosed by the description. Consequently, claims 1-10 also fail to satisfy the requirement prescribed under PCT Article 5 and the requirement of support prescribed under PCT Article 6.

For the same reasons stated above, claims 14-17 depending on one of claims 1-10 fail to satisfy the requirements prescribed under PCT Articles 5 and 6.

Therefore, this international search has been carried out mainly on the compounds of claims 11-13, and no complete search has been carried out on the other compounds.

Form PCT/ISA/210 (extra sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2004217507 A


### Non-patent literature cited in the description


- Comprehensive dictionary on Chemistry. Tokyo Kagaku Dozin Co., Ltd, 1994
- **OYAIZU KENICHI ; YUASA MAKOTO.** *Hyomen,* 2003, vol. 41 (3), 22
- **A.E. BOELRIJK ; G.C. DISMUKES.** *Inorg. Chem.,* 2000, vol. 39, 3020
- **FURUKAWA JUNJI.** Polymer synthesis. Kagaku Dozin Co., Ltd, 20 February 1987, 99-103
- **IWANAMI.** Physical and chemical science dictionary. Iwanami Shoten Publisher, 10 January 1991, 966
- **ANNA L. GAVRILOVA ; BRICE BOSNICH.** *Chem. Rev.,* 2004, vol. 104, 349
- *J. Am. Chem. Soc.,* 1984, vol. 106, 4765-4772
- *J. Am. Chem. Soc.,* 1994, vol. 116, 891-897